# EUROPEAN PATENT APPLICATION

(11) **EP 3 831 381 A1**
(43) Date of publication of application: **09.06.2021**
(21) Application number: 19843904.4
(22) Date of filing: 29.07.2019
(51) Int. Cl.: A61K 31/438, A61K 9/16, A61K 47/02, A61K 47/18, A61K 47/32, A61K 47/38, A61P 5/20

(54) **SOLID DISPERSION OF HYDANTOIN DERIVATIVE**

(30) Priority: 30.07.2018 JP 2018142048
(71) Applicant: CHUGAI SEIYAKU KABUSHIKI KAISHA, Tokyo 115-8543 (JP)
(72) Inventor: KINOSHITA, Ryo, Gotemba-shi, Shizuoka 412-8513 (JP); UETO, Takamitsu, Gotemba-shi, Shizuoka 412-8513 (JP)
(74) Representative: Vossius & Partner Patentanwälte Rechtsanwälte mbB
(86) International application number: PCT/JP2019/029564
(87) International publication number: WO 2020/027011

(57) **Abstract**

The solid dispersion of the present invention contains Compound I having the following structural formula: 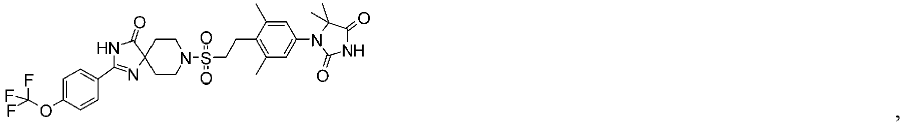 a pharmaceutically acceptable polymer, and a pharmaceutically acceptable cationic species.

## Description

### [Technical Field]

The present invention relates to solid dispersions comprising 1-(3,5-dimethyl-4-(2-((4-oxo-2-(4-(trifluoromethoxy)phenyl)-1,3,8-triazaspiro[4.5]deca-1-en-8-yl)sulfonyl)ethyl)phenyl)-5,5-dimethylimidazolidine-2,4-dione (hereinafter may be referred to as "Compound I"). Specifically, the invention relates to solid dispersions comprising Compound I, pharmaceutically acceptable polymers, and pharmaceutically acceptable cationic species. In addition, the present invention relates to pharmaceutical compositions containing the solid dispersion, methods for producing the solid dispersion, and methods for treating a disease using the pharmaceutical composition.

### [Background Art]

Spiroimidazolone derivatives having a specific structure are known to have a useful parathyroid hormone (PTH)-like effect (Patent Literature (PTL) 1). For example, 1-(3,5-dimethyl-4-(2-((4-oxo-2-(4-(trifluoromethoxy)phenyl)-1,3,8-triazaspiro[4.5]deca-1-en-8-yl)sulfonyl)ethyl)phenyl)-5,5-dimethylimidazolidine-2,4-dione (Compound I) is a compound having a strong PTH-like effect and high metabolic stability, and it can treat conditions that may be treated by a PTH-like effect, including hypoparathyroidism (PTL 2). Furthermore, noninvasive systemic or local exposure to the compound induces bone/cartilage anabolism, enabling methods for prevention, treatment, recovery, and healing acceleration of osteoporosis, bone loss in periodontal disease, alveolar bone defect after tooth extraction, osteoarthritis, articular cartilage defects, adynamic bone disease, achondroplasia, hypochondroplasia, osteomalacia, bone fractures, and such. (PTL 3).

Such a drug is desirably developed into, for example, an orally-available dosage form. However, whether a drug can be developed as an oral preparation depends on how high the bioavailability of the drug is. One of the factors affecting bioavailability is water solubility of the drug. Generally, a poorly water-soluble or insoluble compound shows low bioavailability when administered orally. Improving the bioavailability and oral absorbability of an active ingredient is also important for the active ingredient to exert its medicinal effect stably. In addition, in clinical development, it is generally envisaged that the drug is used in high doses to elevate blood concentration of the drug for improved therapeutic effects; therefore, there is a need for a formulation that is so soluble that it sufficiently dissolves in the digestive tract even when administered in high doses.

To improve drug solubility, solid dispersions containing a poorly soluble compound and a polymer compound have been prepared (Non-Patent Literatures (NPL) 1 to 4).

Furthermore, a document that discloses a solid dispersion containing an ionizable drug, a cationic species, and a dispersion polymer is known (PTL 4). The problem to be solved by the invention described in the above document is enabling easy formation of solid dispersions for a class of compounds for which it is difficult to form a solid dispersion itself, or more specifically, a class of compounds with low aqueous solubility as well as very poor solubility in the volatile solvents used to form spray solutions.

### [Citation List]

### [Patent Literature]

[PTL 1] WO2010/126030
[PTL 2] WO2014/092061
[PTL 3] WO2015/189901
[PTL 4] WO2008/047201

### [Non-Patent Literature]

[NPL 1] J Pharm Sci. 1997 Jan;86(1): 1-12
[NPL 2] AAPS PharmSciTech April 2018, Volume 19, Issue 3, pp 978-990
[NPL 3] Mol. Pharmaceutics 2017, 14, 658-673
[NPL 4] AAPS PharmSciTech January 2018, Volume 19, Issue 1, pp 326-337

### [Summary of Invention]

### [Technical Problem]

As described above, Compound I is useful as a pharmaceutical compound; however, it has poor solubility in water and needs improved water-solubility.

Furthermore, as described above, to increase the blood concentration of the drug and to enhance its therapeutic effect, the drug may be used at a high dose. Therefore, the drug must have solubility high enough to sufficiently dissolve in the digestive tract, even when it is administered at a high dose.

The inventors of the present application discovered that the water solubility of Compound I can be improved by tens of times when using it as a crystal of its salt, for example a besylate salt, compared to when using it as a crystal of its free form. However, it was revealed that while the besylate salt of Compound I showed high water solubility, the bioavailability of the drug when used *in vivo,* i.e. the blood concentration, did not sufficiently increase.

Therefore, regarding a pharmaceutical composition comprising Compound I, a pharmaceutical composition that enables the use of a drug at a high dose, which has higher water solubility and high absorbability even *in vivo,* must be provided.

More specifically, an objective of the present invention is to provide formulations having a high solubility that cannot be achieved by application of conventional arts and having an accompanying high absorbability in the digestive tract, in response to the problem that Compound I has poor water solubility and the demand for its use at a high dose.

### [Solution to Problem]

As a result of dedicated research under this situation, the present inventors prepared a solid dispersion by adding cationic species to the aforementioned Compound I and the polymer, and discovered that this dispersion shows higher solubility and accompanying absorbability in the digestive tract compared to those of conventional solid dispersions containing various polymers and crystals of various salts.

Specifically, the present invention comprises the following:
[1] a solid dispersion which comprises Compound I having the following structural formula: 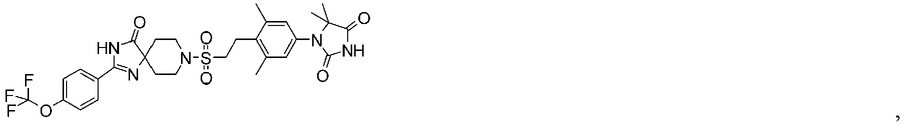 a pharmaceutically acceptable polymer, and a pharmaceutically acceptable cationic species;
[2] the solid dispersion of [1], which comprises Compound I existing in an amorphous form;
[3] the solid dispersion of [1] or [2], wherein the pharmaceutically acceptable polymer is at least one polymer selected from the group consisting of polyvinylpyrrolidone, copovidone, polyvinyl alcohol, cellulosic polymer, and methacrylic acid copolymer;
[4] the solid dispersion of [1] or [2], wherein the pharmaceutically acceptable polymer is at least one polymer selected from the group consisting of polyvinylpyrrolidone, copovidone, polyvinyl alcohol, hydroxypropyl cellulose, hypromellose acetate succinate, and methacrylic acid copolymer LD;
[5] the solid dispersion of any one of [1] to [4], wherein the pharmaceutically acceptable cationic species is at least one cationic species supplied by a base having a pKa value of 11 or higher;
[6] the solid dispersion of any one of [1] to [5], wherein the pharmaceutically acceptable cationic species is at least one selected from the group consisting of a sodium cation, potassium cation, and arginine cation;
[7] the solid dispersion of any one of [1] to [6], wherein the weight ratio of Compound I to the polymer in the composition is about 1:9 to about 1:1;
[8] the solid dispersion of any one of [1] to [7], wherein the weight ratio of Compound I to the polymer in the composition is about 1:2 to about 1:1;
[9] the solid dispersion of any one of [1] to [8], wherein the molar ratio of the cationic species to Compound I in the composition is 0.8 or higher;
[10] the solid dispersion of any one of [1] to [9], which further comprises a surfactant, wherein the surfactant is a pharmaceutically acceptable surfactant;
[11] the solid dispersion of any one of [1] to [10], wherein Compound I is derived from:
   type I crystal of the free form: a crystal of Compound I whose powder X ray diffraction pattern comprises peaks at diffraction angles, represented by 2θ, of 14.4°, 15.3°, 16.6°, and 18.7° (±0.2°); and/or
   type II crystal of the free form: a crystal of Compound I whose powder X ray diffraction pattern comprises peaks at diffraction angles, represented by 2θ, of 7.9°, 13.5°, 15.9°, and 21.8° (±0.2°);
[12] a pharmaceutical composition which comprises the solid dispersion of any one of [1] to [11];
[13] a method for producing a solid dispersion which comprises the steps of:
   (i) providing Compound I having the following structural formula: 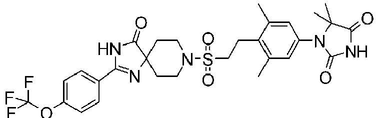 a pharmaceutically acceptable polymer, and a base that yields a pharmaceutically acceptable cationic species;
   (ii) mixing the Compound I, the pharmaceutically acceptable polymer, and the base that yields the pharmaceutically acceptable cationic species; and
   (iii) obtaining a solid dispersion comprising the Compound I, the pharmaceutically acceptable polymer, and the pharmaceutically acceptable cationic species;
[14] the method of [13], wherein the steps (ii) and (iii) comprise the following steps, respectively:
   (ii-a) preparing a mixed solution by mixing the Compound I, the pharmaceutically acceptable polymer, and the base that yields the pharmaceutically acceptable cationic species in a solvent; and
   (iii-a) removing the solvent from the mixed solution obtained in the step (ii-a);
[15] the method of [14], wherein the step (iii-a) comprises the step of removing the solvent and drying by spray drying;
[16] the method of any one of [13] to [15], wherein in the step (i), Compound I is derived from:
   type I crystal of the free form: a crystal of Compound I whose powder X ray diffraction pattern comprises peaks at diffraction angles, represented by 2θ, of 14.4°, 15.3°, 16.6°, and 18.7° (±0.2°); and/or
   type II crystal of the free form: a crystal of Compound I whose powder X ray diffraction pattern comprises peaks at diffraction angles, represented by 2θ, of 7.9°, 13.5°, 15.9°, and 21.8° (±0.2°);
[17] the pharmaceutical composition of [12], wherein the pharmaceutical composition is for preventing or treating hypoparathyroidism, preventing or treating osteoporosis, improving decrease in bone mass in periodontal disease, promoting recovery of alveolar bone defect after tooth extraction, preventing or treating osteoarthritis, promoting recovery of articular cartilage defect, preventing or treating adynamic bone disease, preventing or treating achondroplasia, preventing or treating hypochondroplasia, preventing or treating osteomalacia, or promoting recovery from bone fracture;
[18] a method for preventing or treating hypoparathyroidism, preventing or treating osteoporosis, improving decrease in bone mass in periodontal disease, promoting recovery of alveolar bone defect after tooth extraction, preventing or treating osteoarthritis, promoting recovery of articular cartilage defect, preventing or treating adynamic bone disease, preventing or treating achondroplasia, preventing or treating hypochondroplasia, preventing or treating osteomalacia, or promoting recovery from bone fracture, wherein the method comprises administering the solid dispersion of any one of [1] to [11] or the pharmaceutical composition of [12] to a patient in need thereof;
[19] use of the solid dispersion of any one of [1] to [11] or the pharmaceutical composition of [12] for producing a pharmaceutical composition for preventing or treating hypoparathyroidism, preventing or treating osteoporosis, improving decrease in bone mass in periodontal disease, promoting recovery of alveolar bone defect after tooth extraction, preventing or treating osteoarthritis, promoting recovery of articular cartilage defect, preventing or treating adynamic bone disease, preventing or treating achondroplasia, preventing or treating hypochondroplasia, preventing or treating osteomalacia, or promoting recovery from bone fracture; and
[20] the solid dispersion of any one of [1] to [11] or the pharmaceutical composition of [12] for use in preventing or treating hypoparathyroidism, preventing or treating osteoporosis, improving decrease in bone mass in periodontal disease, promoting recovery of alveolar bone defect after tooth extraction, preventing or treating osteoarthritis, promoting recovery of articular cartilage defect, preventing or treating adynamic bone disease, preventing or treating achondroplasia, preventing or treating hypochondroplasia, preventing or treating osteomalacia, or promoting recovery from bone fracture.

### [Effects of the Invention]

The present invention has enabled great improvement in solubility and the accompanying improvement of absorbability in the digestive tract of the poorly water-soluble compound 1-(3,5-dimethyl-4-(2-((4-oxo-2-(4-(trifluoromethoxy)phenyl)-1,3,8-triazaspiro[4.5]deca-1-en-8-yl)sulfonyl)ethyl)phenyl)-5,5-dimethylimidazolidine-2,4-dione, which has strong PTH-like effects and high metabolic stability.

### [Brief Description of Drawings]

Fig. 1 is a graph showing the results from performing dissolution tests on solid dispersions as measured in Comparative examples 1 and 2. The vertical axis shows the dissolution concentration (µg/mL) of Compound I, and the horizontal axis shows the time (minutes).
Fig. 2 is a graph showing the results from performing dissolution tests on solid dispersions as measured in Comparative examples 3 to 5. The vertical axis shows the dissolution concentration (µg/mL) of Compound I, and the horizontal axis shows the time (minutes).
Fig. 3 is a graph showing the results from performing dissolution tests on solid dispersions as measured in Examples 1 to 3 and Comparative examples 6 and 7. The vertical axis shows the dissolution concentration (µg/mL) of Compound I, and the horizontal axis shows the time (minutes).
Fig. 4 is a graph showing the results from performing dissolution tests on solid dispersions as measured in Examples 4 to 6 and Comparative example 8. The vertical axis shows the dissolution concentration (µg/mL) of Compound I, and the horizontal axis shows the time (minutes).
Fig. 5 is a graph showing the results from performing dissolution tests on solid dispersions as measured in Examples 1, 7, and 8. The vertical axis shows the dissolution concentration (µg/mL) of Compound I, and the horizontal axis shows the time (minutes).
Fig. 6 is a graph showing the results from performing dissolution tests on solid dispersions as measured in Examples 7, 9, and 10. The vertical axis shows the dissolution concentration (µg/mL) of Compound I, and the horizontal axis shows the time (minutes).
Fig. 7 is a graph showing the results from performing dissolution tests on solid dispersions as measured in Examples 11 and 12. The vertical axis shows the dissolution concentration (µg/mL) of Compound I, and the horizontal axis shows the time (minutes).
Fig. 8 is a graph showing the results from performing dissolution tests on solid dispersions as measured in Examples 13 and 14. The vertical axis shows the dissolution concentration (µg/mL) of Compound I, and the horizontal axis shows the time (minutes).
Fig. 9 presents a powder X-ray pattern of the type-I crystal of Compound I in the free form (X-ray source: CuKα₁). The vertical axis shows the intensity, and the horizontal axis shows the angle (2θ).
Fig. 10 presents a powder X-ray pattern of the type-II crystal of Compound I in the free form (X-ray source: CuKα₁). The vertical axis shows the intensity, and the horizontal axis shows the angle (2θ).

### [Description of Embodiments]

### Compound I

The compound contained in the solid dispersion of the present invention is 1-(3,5-dimethyl-4-(2-((4-oxo-2-(4-(trifluoromethoxy)phenyl)-1,3,8-triazaspiro[4.5]deca-1-en-8-yl)sulfonyl)ethyl)phenyl)-5,5-dimethylimidazolidine-2,4-dione (Compound I) having the following structural formula.

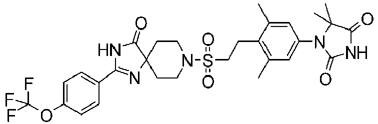

Compound I can be produced, for example, by the method described in Example 3 (Compound 7) of WO2014/092061. In addition, the type-I crystal of the free form of Compound I (Production example 1) has water solubility of less than 1 µg/mL (37°C) at pH 6 to 7, and organic solvent solubility at room temperature of, for example, 80 mg/mL or more in THF, and it is a compound having pKa values of 8 and 10.

That is, Compound I used in the present invention has extremely low water solubility but has relatively high solubility in organic solvents. Therefore, Compound I does not cause any particular problems during the production of solid dispersions using organic solvents.

Compound I that can be used in the present invention includes any isotope of Compound I. An isotope of Compound I is a compound in which at least one atom has been replaced by an atom with the same atomic number (proton number) and a different mass number (sum of the numbers of protons and neutrons). Examples of the isotopes contained in Compound I include a hydrogen atom, a carbon atom, a nitrogen atom, an oxygen atom, a sulfur atom, and a fluorine atom, and their examples are ²H and ³H, ¹³C and ¹⁴C, ¹⁵N, ¹⁷O and ¹⁸O, ³⁵S, and ¹⁸F, respectively. In particular, radioisotopes that decay by emitting radioactivity, such as ³H or ¹⁴C, are useful for examining *in vivo* tissue distribution of pharmaceuticals or compounds. Since a stable isotope does not decay, the existing amount hardly changes, and since there is no radioactivity, it can be used safely. The isotope of Compound I can be converted according to conventional methods by substituting a reagent used for synthesis with the reagent containing the corresponding isotope.

### Polymer

The polymer that can be used in the solid dispersion of the present invention should be pharmaceutically acceptable, and preferably has at least some solubility in an aqueous solution at physiologically relevant pHs (for example, 1 to 8). Almost any polymer that has an aqueoussolubility of at least about 0.1 mg/mL over at least a portion of the pH range of 1 to 8 may be suitable. Examples of the polymer that can be used in the present invention include the following nonionic polymers (neutral polymers) or ionic polymers.

Herein, the phrase "pharmaceutically acceptable" in the phrase "pharmaceutically acceptable polymer" is used in the sense ordinarily used in the art; for example, it means that within the range of ordinary use as a pharmaceutical on a subject receiving the pharmaceutical administration, there is substantially no side effects or toxicity. The same applies to "pharmaceutically acceptable cationic species" and "pharmaceutically acceptable surfactants".

### Nonionic polymer (neutral polymer)

In one embodiment, examples of the polymer that can be used in the present invention include nonionic polymers (neutral polymers). That is, the polymer preferably has substantially no ionic functional group. The expression "has substantially no ionic functional group" means that the number of ionic groups covalently attached to the polymer is less than about 0.05 milliequivalent per gram of polymer. Preferably, the number is less than about 0.02 milliequivalent per gram of neutral polymer. The expression "ionic functional group" means functional groups that are at least about 10% ionized over at least a portion of the physiologically relevant pH range of 1 to 8. Such groups have pKa values of about 0 to 9.

Examples of neutral polymers that can be used in the present invention include neutral non-cellulosic polymers. Examples of such neutral non-cellulosic polymers include the following: vinyl polymers and copolymers having at least one substituent selected from the group comprising hydroxyl, alkylacyloxy, and cyclic amide; vinyl copolymers of at least one hydrophilic hydroxyl-containing repeat unit and at least one hydrophobic alkyl- or aryl-containing repeat unit; polyvinyl alcohols; polyvinyl alcohols having at least a portion of their repeat units in the unhydrolyzed (vinyl acetate) form; polyvinyl alcohol polyvinyl acetate copolymer; polyvinylpyrrolidone; copovidone; acrylate and methacrylate copolymer; polyethylene polyvinyl alcohol copolymer; and polyoxyethylene-polyoxypropylene block copolymer (also referred to as Poloxamer).

Examples of other classes of neutral polymers that can be used in the present invention include neutral cellulosic polymers. The expression "cellulosic" means a cellulose polymer that has been modified by reaction of at least a portion of the hydroxyl groups on the saccharide repeat units with a compound to form an ester or an ether substituent. Examples of such neutral cellulosic polymers include hydroxypropyl methylcellulose acetate (HPMCA), hydroxypropyl methylcellulose (HPMC), hydroxypropyl cellulose (HPC), methylcellulose, hydroxyethyl methylcellulose, hydroxyethyl cellulose, hydroxyethyl cellulose acetate, and hydroxyethyl ethyl cellulose.

In another embodiment, examples of polymers that can be used in the present invention include neutralized acidic polymers. Neutralized acidic polymers are described in more detail in the U.S. Published Patent Application US 2003-0054038, filed June 17, 2002, entitled "Pharmaceutical Compositions of Drugs and Neutralized Acidic Polymers", the relevant disclosure of which is incorporated herein by reference. The expression "acidic polymer" means any polymer that possesses a significant number of acidic moieties. In general, a significant number of acidic moieties would be about 0.05 milliequivalent or more of acidic moieties per gram of polymer. An "acidic moiety" includes any functional group that is sufficiently acidic that, in contact with or dissolving in water, can at least partially donate a hydrogen cation to water and thus increase the hydrogen-ion concentration. This definition includes any functional group or "substituent" (as it is termed when the functional group is covalently attached to a polymer) having a pKa of less than about 10.

The expression "neutralized acidic polymer" means any acidic polymer for which a significant fraction of the "acidic moieties" or "acidic substituents" have been "neutralized"; that is, exist in their deprotonated form. The "degree of neutralization" α of a polymer substituted with monoprotic acids (for example, carboxylic acid) is defined as the fraction of the acidic moieties on the polymer that have been neutralized, i.e., deprotonated by a base.

Typically, in order for an acidic polymer to be considered a "neutralized acidic polymer", α must be at least about 0.01 (or 1%), more preferably at least about 0.1 (10%), still more preferably at least about 0.5 (50%), and most preferably at least 0.9 (90%) (meaning that at least 90% of the acidic moieties have been neutralized).

Examples of the acidic polymers that can be used in the neutralized form in the present invention include cellulose acetate phthalate, cellulose acetate trimellitate, cellulose acetate succinate, methylcellulose phthalate, hydroxymethylcellulose ethyl phthalate, hydroxypropylmethylcellulose phthalate, hydroxypropylmethylcellulose acetate succinate (also referred to as hypromellose acetate succinate) (HPMCAS), hydroxypropylmethyl acetate maleate, hydroxypropylmethyl trimellitate, carboxymethylethylcellulose, polyvinyl butyrate phthalate, polyvinyl alcohol acetate phthalate, methacrylic acid/ethyl acrylate copolymer (preferred mass ratio of 1:99 to 99:1), methacrylic acid/methyl methacrylate copolymer (preferred mass ratio of 1:99 to 99:1), and methacrylic acid copolymer.

The neutralized acidic polymer can be formed by any conventional method known in the art that results in the desired degree of neutralization. Generally, acidic polymers are neutralized through the addition of a sufficient amount of base to a solution or composition containing the acidic polymer. For example, a base may be added to a solution of the acidic polymer resulting in neutralization of the polymer's acidic functional groups. Appropriate bases that can be used for neutralizing acidic polymers include those listed above regarding the cationic species present in the solid dispersion of the present invention.

In one embodiment, examples of the base utilized to neutralize the polymer include the same base used for providing cationic species present in the solid dispersion of the present invention.

### Ionic polymer

In one embodiment, examples of the polymer that can be used in the present invention include an ionic polymer. That is, the polymers substantially have ionic functional groups, and at least about 10% of them are ionized over at least a part of the physiologically relevant pH range of 1 to 8. Ionic polymers are generally classified into acidic polymers and basic polymers in the pH range where they are ionized. The acidic polymer (or enteric polymer) is a polymer having the property of being soluble in a neutral or alkaline solution, and the basic polymer is a polymer having the property of being soluble in an acidic or neutral solution. Specific examples of the acidic polymer include, cellulose acetate phthalate, cellulose acetate trimellitate, cellulose acetate succinate, methylcellulose phthalate, hydroxymethylcellulose ethyl phthalate, hydroxypropylmethylcellulose phthalate, hydroxypropylmethylcellulose acetate succinate (HPMCAS), hydroxypropylmethyl acetate maleate, hydroxypropylmethyl trimellitate, carboxymethylethylcellulose, polyvinyl butyrate phthalate, polyvinyl alcohol acetate phthalate, methacrylic acid/ethyl acrylate copolymer (preferred mass ratio of 1:99 to 99:1), methacrylic acid/methyl methacrylate copolymer (preferred mass ratio of 1:99 to 99:1), and methacrylic acid copolymer; and they are considered to have the property where they start to dissolve rapidly after traveling from the stomach to the upper to mid-small intestine. Specifically, a preferred polymer exhibits the property of dissolving within 120 minutes in a Japanese Pharmacopoeia grade phosphate buffer at pH 6.8. Specific examples of the basic polymer include aminoalkyl methacrylate copolymer E and polyvinyl acetal diethylaminoacetate, which are considered to have the property where they start to dissolve rapidly under acidic conditions in the stomach. Specifically, a preferred polymer exhibits the property of dissolving within 120 minutes in the Japanese Pharmacopoeia 1st fluid for Dissolution Test at pH 1.2.

For the solid dispersion of the present invention, a blend of the above polymers can also be used. That is, the term "polymer" may include blends of polymers in addition to a single type of polymer.

Therefore, in one embodiment, in the solid dispersion of the present invention, the polymer may be selected from the group consisting of neutral polymers, ionic polymers, or mixtures thereof.

Furthermore, in another embodiment, polymers that can be used in the present invention include polymers selected from the group consisting of vinyl polymers and copolymers having at least one substituent selected from the group comprising hydroxyl, alkylacyloxy, and cyclic amide; vinyl copolymers of at least one hydrophilic hydroxyl-containing repeat unit and at least one hydrophobic alkyl- or aryl-containing repeat unit; polyvinyl alcohols; polyvinyl alcohols having at least a portion of their repeat units in the unhydrolyzed (vinyl acetate) form; polyvinyl alcohol polyvinyl acetate copolymer; polyvinylpyrrolidone; copovidone; acrylate and methacrylate copolymer; polyethylene polyvinyl alcohol copolymer; polyoxyethylene-polyoxypropylene block copolymer (also referred to as Poloxamer); hydroxypropyl methylcellulose acetate (HPMCA); hydroxypropyl methylcellulose (HPMC); hydroxypropyl cellulose (HPC); methylcellulose; hydroxyethyl methylcellulose; hydroxyethyl cellulose, hydroxyethyl cellulose acetate; and hydroxyethyl ethyl cellulose which are neutral polymers, cellulose acetate phthalate; cellulose acetate trimellitate; cellulose acetate succinate; methylcellulose phthalate; hydroxymethylcellulose ethyl phthalate; hydroxypropylmethylcellulose phthalate; hydroxypropylmethylcellulose acetate succinate (HPMCAS); hydroxypropylmethyl acetate maleate; hydroxypropylmethyl trimellitate; carboxymethylethylcellulose; polyvinyl butyrate phthalate; polyvinyl alcohol acetate phthalate; methacrylic acid/ethyl acrylate copolymer (preferred mass ratio of 1:99 to 99:1); methacrylic acid/methyl methacrylate copolymer (preferred mass ratio of 1:99 to 99:1); and methacrylic acid copolymer which are acidic polymers, neutralized form of the above acidic polymers, and mixtures thereof.

In one embodiment, preferred polymers that can be used in the present invention include polymers selected from the group consisting of polyvinylpyrrolidone, copovidone, polyvinyl alcohol, cellulosic polymers, and methacrylic acid methacrylic acid copolymers. More preferably, the polymers include those selected from the group consisting of polyvinylpyrrolidone, copovidone, polyvinyl alcohols, hydroxypropyl cellulose, hypromellose acetate succinate, and methacrylic acid copolymer LD. Even more preferably, the polymers include those selected from the group consisting of polyvinylpyrrolidone, copovidone, hydroxypropylcellulose, and methacrylic acid copolymer LD. Particularly preferably, the polymers include polyvinylpyrrolidone.

Among the above, as polyvinylpyrrolidone, specifically, for example, polyvinylpyrrolidone commercially available under the trade names Kollidon 30 and 90F, and such can be used.

As copovidone, specifically, for example, copovidone commercially available under the trade name Kollidon VA64 and such can be used.

As polyvinyl alcohol, specifically, for example, polyvinyl alcohol commercially available under the trade name Parteck MXP and such can be used.

As hydroxypropyl cellulose, specifically, for example, hydroxypropyl cellulose commercially available under the trade name Nisso HPC-SL and such can be used.

As methacrylic acid copolymer LD, specifically, for example, methacrylic acid copolymer LD commercially available under the trade name Eudragit L100-55 and such can be used.

As hydroxypropyl methylcellulose acetate succinate (hypromellose acetate succinate) (HPMCAS), specifically, for example, hydroxypropyl methylcellulose acetate succinate (HPMCAS) commercially available under the trade name AQOAT AS-LF and such can be used.

### Cationic species

The solid dispersion of the present invention contains pharmaceutically acceptable cationic species. The cationic species can be supplied by a base co-dissolved with Compound I.

In the present invention, the "base" preferably has a pKa value greater than that of Compound I. Since the lower pKa value of Compound I is about 8, the bases that can be used in the present invention desirably have a pKa value that is preferably more than about 9, even more preferably more than about 10, and most preferably more than about 11. Here, the term pKa is used in its conventional form. That is, pKa is the negative logarithm of the ionization constant of the acid. Unless otherwise specified, pKa is assumed to be measured in distilled water at 25°C.

Examples of bases that can be used in the present invention include hydroxides such as sodium hydroxide, calcium hydroxide, potassium hydroxide, magnesium hydroxide, ammonium hydroxide, and choline hydroxide; oxides such as magnesium oxide and calcium oxide; amines such as tris(hydroxymethyl)aminomethane, ethanolamine, diethanolamine, N-methylglucamine, glucosamine, ethylenediamine, N,N'-dibenzylethylenediamine, N-benzyl-2-phenethylamine, cyclohexylamine, cyclopentylamine, diethylamine, isopropylamine, diisopropylamine, dodecylamine, and triethylamine; proteins, such as gelatin; and amino acids, such as lysine, arginine, guanine, glycine, and adenine.

Pharmaceutically acceptable cationic species are not particularly limited as long as they are used as pharmaceuticals, and for example, the cationic species are preferably selected from the group consisting of cations of the following: potassium, sodium, calcium, magnesium, aluminum, ammonium, benzathine (N,N'-dibenzylethylenediamine), choline, diethanolamine, ethylenediamine, meglumine (N-methyl glucamine), benethamine (N-benzyl phenethylamine), diethylamine, piperazine, tromethamine (2-amino-2-hydroxymethyl-1,3-propanediol), procaine, and mixtures thereof. More preferably, the cationic species are selected from the group consisting of cations of potassium, sodium, calcium, magnesium, aluminum, and ammonium, and mixtures thereof.

In one embodiment, the cationic species is preferably one or more selected from cationic species supplied by bases having a pKa value of 11 or more. Even more preferably, the cationic species is one or more selected from the group consisting of cations of sodium, potassium, and arginine. Most preferably, the cationic species is sodium cation.

In yet another embodiment, in the solid dispersion of the present invention, examples of a preferred combination of a pharmaceutically acceptable polymer and a cationic species include cases where the polymer is polyvinylpyrrolidone and the cationic species is selected from the group consisting of cations of sodium, potassium, and arginine, and mixtures thereof; and more preferred examples include the following:
the polymer is polyvinylpyrrolidone and the cationic species is a sodium cation;
in another embodiment, the polymer is polyvinylpyrrolidone and the cationic species is a potassium cation; or
in another embodiment, the polymer is polyvinylpyrrolidone and the cationic species is an arginine cation.

Other preferable combinations include cases where the polymer is copovidone and the cationic species is selected from the group consisting of cations of sodium, potassium, and arginine, and mixtures thereof; and more preferred examples include the following: the polymer is copovidone and the cationic species is a sodium cation;
in another embodiment, the polymer is copovidone and the cationic species is a potassium cation; or
in another embodiment, the polymer is copovidone and the cationic species is an arginine cation.

Other preferable combinations include cases where the polymer is polyvinyl alcohol and the cationic species is selected from the group consisting of cations of sodium, potassium, and arginine, and mixtures thereof; and more preferred examples include the following: the polymer is polyvinyl alcohol and the cationic species is a sodium cation;
in another embodiment, the polymer is polyvinyl alcohol and the cationic species is a potassium cation; or
in another embodiment, the polymer is polyvinyl alcohol and the cationic species is an arginine cation.

Other preferable combinations include cases where the polymer is hydroxypropyl cellulose and the cationic species is selected from the group consisting of cations of sodium, potassium, and arginine, and mixtures thereof; and more preferred examples include the following:
the polymer is hydroxypropyl cellulose and the cationic species is a sodium cation;
in another embodiment, the polymer is hydroxypropyl cellulose and the cationic species is a potassium cation; or
in another embodiment, the polymer is hydroxypropyl cellulose and the cationic species is an arginine cation.

Other preferable combinations include cases where the polymer is hypromellose acetate succinate and the cationic species is selected from the group consisting of cations of sodium, potassium, and arginine, and mixtures thereof; and more preferred examples include the following:
the polymer is hypromellose acetate succinate and the cationic species is a sodium cation;
in another embodiment, the polymer is hypromellose acetate succinate and the cationic species is a potassium cation; or
in another embodiment, the polymer is hypromellose acetate succinate and the cationic species is an arginine cation.

Other preferable combinations include cases where the polymer is methacrylic acid copolymer LD and the cationic species is selected from the group consisting of cations of sodium, potassium, and arginine, and mixtures thereof; and more preferred examples include the following:
the polymer is methacrylic acid copolymer LD and the cationic species is a sodium cation;
in another embodiment, the polymer is methacrylic acid copolymer LD and the cationic species is a potassium cation; or
in another embodiment, the polymer is methacrylic acid copolymer LD and the cationic species is an arginine cation.

### Surfactant

The solid dispersion of the present invention and the pharmaceutical composition containing the same may further contain a surfactant. The surfactant may be co-dispersed in the solid dispersion of the present invention, or it may be mixed (combined) outside of the solid dispersion in the same manner as the other additives. Surfactants that can be used in the present invention are pharmaceutically acceptable surfactants.

In the present invention, "surfactant" means a substance having both hydrophilic and hydrophobic groups in a molecule, and includes ionic surfactants and non-ionic surfactants.

An ionic surfactant refers to an ionic surfactant that is electrolyzed and forms ions (charged atoms or groups of atoms) when dissolved in water. Ionic surfactants are further classified into anionic surfactants, cationic surfactants, and amphoteric surfactants depending on the charge of the generated ions.

Examples of nonionic surfactants include sugar ester type surfactants such as sorbitan fatty acid ester (C12-18), polyoxyethylene (POE) sorbitan fatty acid ester (C12-18), and sucrose fatty acid ester; fatty acid ester types such as POE fatty acid ester (C12-18), POE resin acid ester, and POE fatty acid diester (C12-18); alcohol types such as POE alkyl ether (C12-18); alkylphenol type surfactants such as POE alkyl (C8-12) phenyl ether, POE dialkyl (C8-12) phenyl ether, and POE alkyl (C8-12) phenyl ether formalin condensate; polyoxyethylene-polyoxypropylene block polymer type surfactants such as polyoxyethylene-polyoxypropylene block polymer and alkyl (C12-18) polyoxyethylene-polyoxypropylene block polymer ether; alkylamine types such as POE alkyl alkylamine (C12-18) and POE fatty acid amide (C12-18); bisphenol type surfactants such as POE fatty acid bisphenyl ether; polyaromatic ring type surfactants such as polyoxyalkylene (POA) benzyl phenyl (or phenyl phenyl) ether and POA styryl phenyl (or phenyl phenyl) ether; POE ether and ester type silicon and fluorine surfactants; and plant oil type surfactants such as POE castor oil and POE hydrogenated castor oil.

Non-ionic surfactants preferably include polyoxyl 40 stearate, sorbitan trioleate, polyoxyethylene (105) polyoxypropylene (5) glycol, polyoxyethylene hydrogenated castor oil 60, polyoxyl 35 castor oil, lauromacrogol, and tocopherol polyethylene glycol succinate (TPGS).

Examples of anionic surfactants include sulfate type surfactants such as alkyl sulfate (C12-18), POE alkyl ether sulfate (C12-18), POE alkylphenyl ether sulfate (C12-18), POE benzyl (or styryl) phenyl (or phenyl phenyl) ether sulfate, polyoxyethylene, and polyoxypropylene block polymer sulfate; sulfonate type surfactants such as paraffin (alkane) sulfonate (C12-22), α-olefin sulfonate (C14-16), dialkylsulfosuccinate (C8-12), alkylbenzene sulfonate (C12), mono- or dialkyl (C3-6) naphthalenesulfonate, naphthalene sulfonate-formalin condensate, alkyl (C8-12) diphenyl ether disulfonate, lignin sulfonate, POE alkyl (C8-12) phenyl ether sulfonate, and POE alkyl (C12-18) ether sulfosuccinic acid half-ester; carboxylic acid type surfactants such as fatty acid salt (C12-18), N-acyl amino acid salt (C12-18), and resin acid salt; and phosphate type surfactants such as POE alkyl (C12-18) ether phosphate, POE mono- or dialkyl (C8-12) phenyl ether phosphate, POE benzyl (or styryl) phenyl (or phenyl phenyl) ether phosphate, polyoxyethylene-polyoxypropylene block polymer, and alkyl (C8-12) phosphate.

Anionic surfactants preferably include monoalkyl sulfates such as sodium lauryl sulfate, sodium tetradecyl sulfate, sodium hexadecyl sulfate, and sodium octadecyl sulfate; dioctyl sodium sulfosuccinate; sodium lauroylsarcosine; and sodium dodecylbenzene sulfonate.

In the present invention, two or more surfactants may be used upon combining them at an appropriate ratio.

More preferable surfactants include those selected from the group consisting of monoalkyl sulfates, sorbitan trioleate, polyoxyethylene (105) polyoxypropylene (5) glycol, polyoxyethylene hydrogenated castor oil 60, polyoxyl 35 castor oil, dioctyl sodium sulfosuccinate, sodium lauroylsarcosine, sodium dodecylbenzene sulfonate, tocopherol polyethylene glycol succinate (TPGS), and mixtures thereof.

Even more preferable surfactants are selected from the group consisting of sodium lauryl sulfate, polyoxyethylene hydrogenated castor oil 60, tocopherol polyethylene glycol succinate (TPGS), and mixtures thereof, and a particularly preferable example of the surfactant is sodium lauryl sulfate.

### Solid dispersion

The solid dispersion of the present invention comprises Compound I, a pharmaceutically acceptable polymer, and a pharmaceutically acceptable cationic species.

Herein, "solid dispersion" means that at least a portion of the drug is dispersed in the polymer. Such solid dispersions are sometimes referred to in the art as "molecular dispersions" or "solid solutions" of drug in the polymer.

Herein, "crystal" means a particular solid form of a compound that shows long-range order in three dimensions. The term "amorphous" refers to a material not having long-range three-dimensional order, which not only includes materials which have essentially no order, but also materials that can have some order (order that is less than three dimensions and/or order over a very short distance). Another term for an amorphous form of a material is the "Non-crystalline" form of the material.

Compound I can take either a crystalline form or an amorphous form. In the solid dispersion, preferably at least about 90% by weight and more preferably at least about 95% by weight of the total weight of Compound I is amorphous. In other words, in the solid dispersion, desirably, the amount of Compound I in crystalline form preferably does not exceed about 10% by weight, and more preferably does not exceed about 5% by weight of the total weight of Compound I.

The amount of crystalline and amorphous Compound I can be determined by techniques known in the art, such as powder X-ray diffraction (XRPD), scanning electron microscope (SEM) analysis, solid-state NMR, or thermal techniques including differential scanning calorimetry (DSC), or any other standard quantitative measurement methods.

The amorphous Compound I in the solid dispersion can exist in several phases. For example, it may exist as a single phase of Compound I, or as a solid solution uniformly dispersed in the entire polymer, or any combination of these states or a state that lies between them. Preferably, Compound I and the polymer exist in the form of a solid solution. A solid solution is thermodynamically stable, and Compound I is dispersed in the polymer at the molecular level in this solution, i.e., Compound I is dissolved.

When the amorphous Compound I and the polymer have glass transition temperatures (Tg) that differ by more than about 20°C, whether Compound I exists in the form of a solid solution in a polymer can be determined by measuring the Tg of the solid dispersion. Tg as used herein is a characteristic temperature at which a glass-like material, upon gradual heating, undergoes a relatively rapid (i.e., in 10-100 seconds) physical change from a glass state to a rubber state. The Tg of an amorphous material such as a polymer or a solid dispersion can be measured by several techniques such as dynamic mechanical analyzer (DMA), dilatometer, dielectric analyzer, and DSC. Although the exact value measured by each technique can vary somewhat, it is usually within the range of 10°C to 30°C of each other. When a solid dispersion shows a single Tg, the amount of Compound I in a single amorphous phase in the dispersion is generally less than about 10% by weight, which supports that the dispersion is substantially homogenous.

Preferably, the solid dispersion shows at least one Tg different from the respective Tg of Compound I and the polymer used, and at least a part of Compound I and the polymer exist as a solid solution.

Furthermore, from the aspect of physical stability, a high Tg is desirable for the solid dispersion. According to Friesen, 5, Mol. Pharm. (2008), and others, the time required for 5% phase separation to take place in the solid dispersion increases about ten times as the Tg increases by 10°C, and when the Tg of the solid dispersion is higher than the storage temperature by about 30°C or more, 5% phase separation will not occur for at least two years at that storage temperature (it can be regarded as physically stable). For example, assuming a storage temperature of 30°C, if the Tg of the solid dispersion is at least 60°C or higher, preferably 80°C or higher, and more preferably 100°C or higher, physical stability can be expected to be maintained during storage for a long time.

In the solid dispersion of the present invention, the content ratio of Compound I and the polymer depends on the properties of the polymer and is not particularly limited, and Compound I:polymer (weight ratio) preferably varies widely from about 1:100 to about 3:1 (for example, when only Compound I and the polymer are the constituents, the content of Compound I becomes 1% to 75% by weight). The range of Compound I:polymer (weight ratio) is more preferably about 1:9 to about 2:1, still more preferably about 1:9 to about 1:1, even more preferably about 1:5 to about 1:1, and particularly preferably, about 1:2 to about 1:1.

Herein, "about" means preferably a range of ±10%, more preferably a range of ±5%, and even more preferably a range of ±1% of the amount described.

In the solid dispersion of the present invention, the content ratio between Compound I and the cationic species is not particularly limited, and for example, the molar ratio of the aforementioned cationic species with respect to Compound I in the composition is preferably 0.8 or more, and the molar ratio of Compound I:cationic species is more preferably about 1:0.8 to 1:10, even more preferably about 1:1 to 1:5, and particularly preferably about 1:1 to 1:2. That is, preferably, the cationic species is present in a slightly smaller, equal, or excess amount in terms of molar ratio with respect to Compound I.

The solid dispersion of the present invention preferably contains at least about 1% by weight of Compound I relative to the total weight of the solid dispersion. In another aspect, more preferably, the solid dispersion desirably contains at least about 5% by weight, at least about 10% by weight, at least about 15% by weight, at least about 20% by weight, at least about 25% by weight, at least about 30% by weight, at least about 35% by weight, at least about 40% by weight, at least about 45% by weight, or at least about 50% by weight of Compound I relative to the total weight of the solid dispersion.

When the solid dispersion of the present invention further contains a surfactant, the content ratio of Compound I and the surfactant depends on the properties of the surfactant and is not particularly limited, and Compound I: surfactant (weight ratio) preferably varies widely from about 1:5 to about 5:1. Compound I:surfactant (weight ratio) is more preferably in the range of about 1:3 to about 3:1, and even more preferably in the range of about 1:2 to about 2:1.

In yet another embodiment, the solid dispersion of the present invention may be composed of a plurality of particles. Each of the aforementioned particles include Compound I, a pharmaceutically acceptable polymer, and a pharmaceutically acceptable cationic species. This is different from a simple physical mixture of Compound I particles and polymer particles. In a preferred embodiment, the solid dispersion is composed of a plurality of particles, and each particle comprises Compound I, a pharmaceutically acceptable cationic species, and a pharmaceutically acceptable polymer. In this case, preferably, Compound I, the cationic species, and the polymer are in the form of a solid solution.

### Method for producing a solid dispersion

The method for producing the solid dispersion of the present invention comprises the following steps:
(i) providing Compound I having the following structural formula: 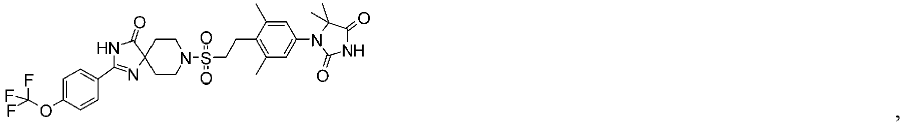 a pharmaceutically acceptable polymer, and a base that yields a pharmaceutically acceptable cationic species;
(ii) mixing the Compound I, the pharmaceutically acceptable polymer, and the base that yields the pharmaceutically acceptable cationic species; and
(iii) obtaining a solid dispersion comprising the Compound I, the pharmaceutically acceptable polymer, and the pharmaceutically acceptable cationic species.

The aforementioned steps (ii) and (iii) preferably include the following steps, respectively:
(ii-a) preparing a mixed solution by mixing into a solvent the Compound I, the pharmaceutically acceptable polymer, and the base that yields the pharmaceutically acceptable cationic species; and
(iii-a) removing the solvent from the mixed solution obtained in step (ii-a).

The aforementioned step (iii-a) preferably includes the step of removing the solvent and drying by spray drying.

In the solid dispersion of the present invention, the solid dispersion only needs to include at least the three components, Compound I, a pharmaceutically acceptable polymer, and a pharmaceutically acceptable cationic species, and in the above-mentioned step (i) of the method of producing a solid dispersion, the form in which Compound I is provided is not particularly limited.

For example, Compound I can be supplied as its free form into a solid dispersion. Alternatively, Compound I can be supplied as a pharmaceutically acceptable salt, or a hydrate or solvate thereof.

Examples of salts include inorganic acid salts, organic acid salts, inorganic base salts, organic base salts, and acidic or basic amino acid salts.

Preferred examples of inorganic acid salts include hydrochloride, hydrobromide, sulfate, nitrate, and phosphate. Preferred examples of organic acid salts include acetate, succinate, fumarate, maleate, tartrate, citrate, lactate, stearate, benzoate, methanesulfonate, benzenesulfonate, and p-toluenesulfonate. Preferred examples of inorganic base salts include alkali metal salts such as sodium salt and potassium salt, alkaline earth metal salts such as calcium salt and magnesium salt, aluminum salt, and ammonium salt. Preferred examples of organic base salts include diethylamine salt, diethanolamine salt, meglumine salt, and N,N-dibenzylethylene diamine salt. Preferred examples of acidic amino acid salts include aspartate and glutamate, and preferred examples of basic amino acid salts include arginine salt, lysine salt, and ornithine salt.

Compound I used in the present invention may absorb water and become a hydrate by being left in the atmosphere, and such a hydrate may also be used as a raw material of Compound I.

In addition, Compound I used in the present invention may absorb some other solvent to form a solvate, and such a solvate may also be used as a raw material of Compound I.

Of these, Compound I is preferably supplied as its free form.

Such Compound I can be obtained by the method described in WO2014/092061 and such.

Furthermore, in the present invention, Compound I may be supplied in a crystalline or amorphous form. When crystalline, polymorphs are included in the crystals of Compound I; however, either of the crystal forms may be supplied alone, or a mixture of crystal forms may be supplied.

As described in the Examples herein, the present inventors discovered that the free form of Compound I used in the present invention has some polymorphisms. Examples of crystalline polymorphs include crystals characterized by a powder X-ray diffraction pattern having peaks at diffraction angles (2θ) of 14.4°, 15.3°, 16.6°, and 18.7° (±0.2°) (hereinafter, referred to as "type I crystals") and crystals characterized by a powder X-ray diffraction pattern having peaks at diffraction angles (2θ) of 7.9°, 13.5°, 15.9°, and 21.8° (±0.2°) (hereinafter referred to as "type II crystals").

As the pharmaceutically acceptable cationic species, the aforementioned cationic species can be used, and the base yielding a pharmaceutically acceptable cationic species can be supplied in the form of a solid or as a solution containing the base in the aforementioned step (i). As the preferred base and cationic species, those similar to the aforementioned base and cationic species can be preferably used.

The above-mentioned polymer may be used as a pharmaceutically acceptable polymer, and the polymer may be supplied to the above-mentioned step (i) directly as a solid or by dissolving it in a solvent in advance. As the preferred polymer, those similar to the aforementioned polymers can be preferably used.

When the solid dispersion of the present invention further contains a surfactant, the aforementioned surfactants can be used, and in the aforementioned step (i), the surfactant can be supplied directly as a solid or by dissolving it in a solvent in advance. As the preferred surfactant, those similar to the aforementioned surfactants can be preferably used.

In the method for producing a solid dispersion of the present invention, any conventional method, preferably, in which at least a portion of Compound I is in an amorphous state can be used. For example, when a solid dispersion is formed from Compound I, the pharmaceutically acceptable polymer, and the base yielding the pharmaceutically acceptable cationic species, as the solvent process, nonsolvent precipitation, freeze drying, spray coating, spray drying, and such can be used. As the melting process, hot-melt extrusion (HME) or the like can be used. As a mechanical process, mixing and crushing, or such can be used.

Generally, the solvent process comprises the step of dissolving at least a portion of the drug, at least a portion of one or more polymer components, and at least a portion of the base providing one or more cationic species in a common solvent. The term "solvent" is used broadly and includes a mixture of solvents. Here, "common" means that the solvent (which may be a mixture of compounds) dissolves at least a portion of the drug and the polymer(s). Preferably, the solvent essentially dissolves all of the drug, all of the polymer, and all of the base.

A solvent suitable for solvent treatment may be any compound in which drugs, polymers, and bases are mutually soluble. Preferably, the solvent is also volatile and has a boiling point of 150°C or lower. Furthermore, the solvent should have relatively low toxicity and should be removed from the solid dispersion to a level that is acceptable according to the guidelines of the International Council for Harmonization of Technical Requirements for Pharmaceuticals for Human Use (ICH). Removal of the solvent to this level may require a subsequent processing step such as tray-drying. Preferred solvents include alcohols such as methanol, ethanol, n-propanol, iso-propanol, and butanol; ketones such as acetone, methyl ethyl ketone, and methyl iso-butyl ketone; esters such as ethyl acetate and propyl acetate; and various other solvents, such as acetonitrile, methylene chloride, toluene, 1,1,1-trichloroethane, and tetrahydrofuran. Low-volatility solvents such as dimethylacetamide or dimethylsulfoxide can also be used in small amounts in mixtures with volatile solvents. A mixture of solvents such as 50% methanol and 50% acetone can also be used in the same manner as a mixture with water. Preferred solvents are ethanol, methanol, acetone, tetrahydrofuran, ethyl acetate, mixtures of these with water, and mixtures thereof. Furthermore, the present Compound I dissolves well in acetone and tetrahydrofuran, and for example, its solubility is 50 mg/mL or more in a mixed solution of acetone and water (volume ratio 95:5), 80 mg/mL or more in tetrahydrofuran, and 100 mg/mL or more in a mixed solution of tetrahydrofuran and methanol (volume ratio 95:5). In particular, tetrahydrofuran and its mixed liquids can be referred to as preferred solvents.

When at least a portion of each of Compound I, the polymer, and the base providing the cationic species are dissolved, the solvent is removed by evaporation or by mixing with a nonsolvent. Exemplary methods include spray drying, spray coating (pan coating, fluidized bed coating, etc.), freeze drying, and precipitation by rapid mixing of a solution of drugs, polymers, and bases with CO₂, hexane, heptane, water at an appropriate pH, or any other nonsolvent. It is preferable to obtain a substantially homogeneous solid dispersion after removing the solvent. In order to achieve this goal, it is generally desirable to quickly remove the solvent from the solution such as in a process where the solution is atomized and the drug and the polymer rapidly solidify.

### Spray drying method

The solvent can be removed by spray drying. The term "spray drying" is used conventionally, and broadly refers to a method that involves breaking up a liquid mixture into small droplets (atomization) and rapidly removing solvent from the mixture in a spray drying apparatus where there is a strong driving force for evaporation of solvent from the droplets. The spray drying method and spray drying apparatus are described generally in Perry's Chemical Engineers' Handbook, pages 20-54 to 20-57 (6th edition, 1984). More details on the spray drying method and apparatus are outlined in Marshall, "Atomization and Spray-Drying," 50 Chem. Eng. Prog. Monogr. Series 2 (1954) and Masters, Spray Drying Handbook (4th edition, 1985). The strong driving force for solvent evaporation is generally provided by maintaining the partial pressure of the solvent in the spray drying apparatus well below the vapor pressure of the solvent at the temperature of the drying droplets. This is accomplished by (1) maintaining the pressure in the spray drying apparatus at a partial vacuum (for example, 0.01 to 0.50 atm); or (2) mixing the liquid droplets with a warm drying gas; or (3) both (1) and (2). Furthermore, at least a portion of the heat necessary for solvent evaporation may be provided by heating the spray solution.

The solvent-bearing feed can be spray-dried under a wide variety of conditions, and yet still yield solid dispersions with acceptable properties. For example, various types of nozzles can be used to atomize the spray solution, thereby introducing the spray solution into the spray dry chamber as a collection of small droplets. Essentially any type of nozzle can be used for spraying the solution as long as the droplets that are formed are sufficiently small that they dry sufficiently (by evaporation of the solvent) such that they do not stick to or coat the spray drying chamber wall.

The maximum droplet size varies widely depending on the size, shape, and flow pattern in the spray dryer, but generally, the diameter of the droplets should be less than about 500 µm when they exit the nozzle. Examples of the types of nozzles that can be used to form solid dispersions include two-fluid nozzles, fountain-type nozzles, flat fan-type nozzles, pressure nozzles, and rotary atomizers. In a preferred embodiment, a pressure nozzle is used. The details are disclosed in U.S. Published Patent Application No. US2003/0185893, filed January 24, 2003, the disclosure of which is incorporated herein by reference.

The spray solution can be delivered to the spray nozzle or nozzles at a wide range of temperatures and flow rates. Generally, the spray solution temperature may be at any range from just above the freezing point of the solvent to about 20°C above its ambient pressure boiling point (by pressurizing the solution). In some cases, it can be even higher. Spray solution flow rates to the spray nozzle can vary over a wide range depending on the type of nozzle, spray dryer size, and spray dry conditions (for example, the inlet temperature and flow rate of the drying gas). Generally, energy for evaporating solvent from a spray solution in a spray drying method comes primarily from the drying gas.

The drying gas can, in principle, be essentially any gas, but for safety reasons and to minimize undesirable oxidation of drugs or other substances in solid dispersions, an inert gas such as nitrogen, nitrogen-enriched air, or argon is used. The drying gas is typically introduced into the drying chamber at a temperature between about 60°C and about 240°C.

The large surface-to-volume ratio of the droplets and the large driving force for evaporation of solvent leads to rapid solidification times for the droplets. Solidification times should be less than about 20 seconds, preferably less than about ten seconds, and more preferably less than one second. This rapid solidification is often critical for the particles to maintain a uniform and homogeneous dispersion, instead of separating into a drug-rich phase and a polymer-rich phase.

Following solidification, the solid powder typically stays in the spray drying chamber for about 5 to 60 seconds, further evaporating solvent from the solid powder. The final solvent content of the solid dispersion as it exits the dryer should be low, since this reduces the mobility of the drug molecules in the solid dispersion, thereby improving its stability. Generally, the solvent content of the solid dispersion as it leaves the spray drying chamber should be less than 10% by weight, preferably less than 2% by weight, and more preferably less than 1% by weight.

Following formation, to remove residual solvent, the solid dispersion may be dried using suitable drying methods. Examples include tray drying, vacuum drying, fluid bed drying, microwave drying, belt drying, rotary drying, and other drying methods known in the art. The preferred secondary drying method is vacuum drying, tray drying, or such. To minimize chemical degradation during drying, the drying can be performed under an inert gas such as nitrogen or under vacuum.

Solid dispersions are usually in the form of small particles. The volumetric mean diameter of the particles may be less than 500 µm in diameter, or less than 100 µm in diameter, less than 50 µm in diameter, or less than 25 µm in diameter. When the solid dispersion is formed by spray drying, the resulting dispersion is in the form of such small particles.

### Spray coating method

In another embodiment, the solvent is removed by spraying the solvent-bearing feed solution onto a seed core. The seed core can be manufactured from any suitable material such as starch, microcrystalline cellulose, sugar, or wax, by any known method such as melt- or spray-congealing, extrusion/spheronization, granulation, and spray drying. The feed solution can be sprayed onto such a seed core using a coating device or a granulation device known in the field of pharmaceuticals. Examples include pan coater (for example, Hi-Coater available from Freund Corp. of Tokyo, Japan, and Accela-Cota available from Manesty of Liverpool, UK), fluid bed coater (for example, Wurster coaters or top-sprayers available from Glatt Air Technologies of Ramsey, New Jersey, and Niro Pharma Systems of Bubendorf, Switzerland), and rotary granulator (for example, CF-Granulator available from Freund Corp). During this method, the seed core is coated with the feed solution and the solvent is evaporated, resulting in a coating containing the solid dispersion. The particles so formed will have a density similar to that of the seed core, and the processing and handling of the composition may improve.

### Hot melt extrusion (HME) method

Melting process can be carried out using a conventional stirrer with a heat source, a kneading machine, and such. Furthermore, those having a structure that allows adding pressure to its interior are more preferred. For example, an extrusion machine having a screw in a cylinder (for example, a single-screw extrusion machine, a twin-screw extrusion machine, etc.), and an injection device of an injection molding machine (for example, a twin-screw type extruder) can be used. Among them, the injection device of the twin-screw type extruder is preferred (for example, Pharma16 twin-screw extruder available from Thermo Fisher Scientific of Massachusetts, USA).

In this case, drugs, polymers, and other components such as plasticizers and surfactants, as needed, are placed from the hopper of these devices to the inside of the device that is maintained at an appropriate heating and melting temperature, and the screw is rotated to melt and evenly mix the solid substance. Alternatively, if necessary, they may be mixed preparatorily before being placed into the device. The conditions set for pressure, temperature, powder supply rate, die diameter, screw shape, number of screw rotations, and such in the manufacturing method of the present invention vary depending on the type of drugs, polymers, and such or the model of the device used, and it is important to combine them so that the temperature is kept below the decomposition temperature of each component, and these settings are desirably changed according to the characteristics of the product of interest. Melting and kneading may be followed by treatments such as cooling, solidification, and further pulverization as necessary, to obtain the solid dispersion of the present invention.

When the solid dispersion of the present invention further comprises a surfactant, the surfactant may be co-dispersed in the solid dispersion of the present invention, or it may be mixed (combined) outside of the solid dispersion in the same manner as the other additives. When co-dispersing the surfactant, for example, in the aforementioned solvent process, the surfactant is dissolved in the solvent together with Compound I, polymer, and base, and the solvent is removed by spray drying, which may result in the desired solid dispersion. In addition, when the surfactant is mixed by coating the outside of the solid dispersion, for example, a conventional blender/mixer or such is used to physically mix the surfactant with the obtained solid dispersion to prepare the desired physical mixture.

### Formulation

The solid dispersion obtained as described above can be used as a pharmaceutical composition directly or after being mixed with any other components used in the field of pharmaceutical formulation.

The other components are not particularly limited as long as they are pharmaceutically acceptable, and include, for example, excipients, and as needed, binders, disintegrators, lubricants, colorants, flavoring agents, stabilizers, emulsifiers, absorption accelerators, surfactants, pH adjusters, preservatives, and antioxidants.

The pharmaceutical composition comprising the solid dispersion of the present invention can be formulated by commonly used methods into tablets, powders, fine granules, granules, coated tablets, capsules, troches, suppositories, ointments, ophthalmic ointments, cataplasms, and the like. In general, it is preferably formulated as an oral preparation that can be administered orally. For the formulations, ordinarily used excipients, binders, lubricants, colorants, correctives, and as necessary, stabilizers, emulsifiers, absorption enhancers, surfactants, pH adjusters, preservatives, antioxidants, and such can be used. Formulations are performed by conventional methods by combining components generally used as raw materials for pharmaceutical preparations.

For example, to produce oral formulations, excipients, and further as necessary, binders, disintegrators, lubricants, colorants, correctives, stabilizing agents, emulsifiers, absorption enhancers, surfactants, pH adjusters, preservatives, antioxidants, and such are added to the solid dispersion of the present invention, and then, this is made into powders, fine granules, granules, tablets, coated tablets, capsules, and such by ordinary methods.

Examples of excipients include lactose, corn starch, white sugar, glucose, mannitol, sorbitol, starch, crystalline cellulose, silicon dioxide, and magnesium aluminometasilicate.

Examples of binders include polyvinyl alcohol, polyvinyl ether, methylcellulose, ethyl cellulose, gum arabic, tragacanth, gelatin, shellac, hydroxypropyl methylcellulose, hydroxypropyl cellulose, polyvinylpyrrolidone, and polypropylene glycol-polyoxyethylene block polymer.

Examples of disintegrators include starch, pregelatinized starch, agar, gelatin powder, crystalline cellulose, calcium carbonate, sodium chloride, sodium bicarbonate, calcium citrate, silicic anhydride, dextrin, pectin, carmellose, carmellose calcium, croscarmellose sodium, low-substituted hydroxypropyl cellulose, and sodium carboxymethyl starch.

Examples of lubricants include magnesium stearate, calcium stearate, talc, polyethylene glycol, silica, and hydrogenated vegetable oil.

As the colorants, those approved as additives to pharmaceuticals are used. As correctives, cocoa powder, peppermint camphor, empasm, mentha oil, borneol, and powdered cinnamon bark are used.

Obviously, these tablets and granules may be sugar-coated or otherwise coated appropriately as necessary.

As described in WO2014/092061and WO2015/189901, Compound I has strong PTH-like effects and high metabolic stability, and it enables treatment of pathological conditions including hypoparathyroidism which may be treated by PTH-like effects. Also, through noninvasive whole body exposure or topical exposure, it induces bone/cartilage anabolism, and it can also provide methods for preventing, treating, recovering from, and promoting healing of osteoporosis, reduced bone mass in periodontal disease, alveolar bone defect after tooth extraction, osteoarthritis, articular cartilage defects, aplastic osteopathy, achondroplasia, hypochondroplasia, osteomalacia, bone fractures, and such.

The dosage of the pharmaceutical composition of the present invention can be appropriately selected according to the degree of symptoms, age, sex, body weight, administration form, type of salt, specific type of disease, and the like.

The dosage differs significantly depending on the patient's disease type, symptom level, patient's age, gender, and sensitivity to the drug, etc., but usually the dose for an adult is about 0.03 to 1000 mg per day, preferably 0.1 to 500mg per day, or more preferably 0.1 to 100 mg per day, administered at once or in several portions in a day.

### Dissolution test

In a preferred embodiment, the solid dispersion of the present invention provides good dissolution in *in vitro* dissolution tests. In particular, improved solubility in an *in vitro* dissolution test which uses an artificial simulated intestinal fluid (fasted-state simulated intestinal fluid: FaSSIF) as a test solution is known to correlate with good *in vivo* bioavailability (that is, an increase in blood concentration). For example, Takano, 23, Pharm. Res. (2006) and others have reported that digestive tract absorption rates (Fa) upon oral administration, calculated for twelve model drugs from the dissolution profiles in the *in vitro* dissolution tests using FaSSIF mentioned above, show good correlation with Fa obtained from human *in vivo* tests.

A suitable FaSSIF is, for example, an aqueous solution containing 28.7 mM potassium dihydrogen phosphate (KH₂PO₄), 103.3 mM KCl, 3 mM sodium taurocholate, and 0.8 mM L-α-phosphatidylcholine, and adjusted to pH 6.5 using NaOH.

An *in vitro* dissolution test can be carried out, for example, by applying an excess amount of the composition of the present invention to an FaSSIF solution heated to 37°C, sequentially sampling while stirring using a paddle-type dissolution tester (for example, VK7010 available from Varian Medical Systems, California, USA), filtering the samples through a 0.45 µm PVDF filter, and then quantifying the concentration of the compound in the resulting filtrate by HPLC.

In one embodiment, the composition of the present invention provides good drug dissolution in an *in vitro* dissolution test. This is at least two times the dissolution provided by the control composition during the test time of the *in vitro* dissolution test (for example maximum of 120 minutes). For example, when the dissolution provided by the control composition is at most 50 µg/mL in 120 minutes, the composition of the present invention provides at most 100 µg/mL dissolution for at least 120 minutes. More preferably, the composition of the present invention provides drug dissolution that is at least three times, or in some cases at least five times or more, the dissolution provided by the control composition. The composition of the present invention is a solid dispersion comprising a drug, a polymer, and a cationic species, and the control composition includes a crystalline or amorphous form of a single drug, a solid dispersion containing only a drug and a polymer, a dispersion containing only a drug and a cationic species, and the like. In addition, when the composition of the present invention comprises a surfactant or the like that affects the solubility of the drug, the above-mentioned control compositions containing the same components in the same amount are included. Furthermore, in addition to the maximum drug dissolution concentration within the test time of the *in vitro* dissolution test, comparing the drug concentration at the end of the test (for example, 120 minutes) is also preferred. This is because, when assuming a good *in vivo* bioavailability, considering retention and such in the digestive tract, maintaining high dissolution even for a relatively long time is preferred.

### [Example]

Herein below, the present disclosure will be described in more detail with reference to Examples. However, the present disclosure can be realized in various embodiments and is not to be construed as being limited to the Examples described herein. In the Examples and Comparative examples described below, Compound I used as the starting material is a type I crystal or a type II crystal of its free form. In Examples 1 to 12 and Comparative examples 1 to 8, the type II crystals were used, and in Examples 13 and 14 the type I crystals were used.

### Example 1

This example describes the formation of a solid dispersion containing Compound I, polyvinylpyrrolidone as a polymer, and sodium cation as a cationic species. Polyvinylpyrrolidone is a polymer of N-vinyl-2-pyrrolidone and is a nonionic polymer.

### [Example 1]

Compound I and polyvinylpyrrolidone (Kollidon 30, BASF) at a weight ratio of 1:2 (compound 33.3% and polymer 66.7%) were dissolved at room temperature in a mixture of ethanol (Junsei Chemical) and an aqueous 2 M NaOH solution (Wako Pure Chemicals) (volume ratio of 95:5) at about 12% solid content concentration (4% in terms of Compound I concentration). Next, the obtained solution was spray-dried using a small spray dryer B-290 (BUCHI) to obtain a solid dispersion (Compound I: sodium molar ratio of 1:1.6).

For example, when preparing a 2.4-g batch, 19 mL of ethanol and 1 mL of an aqueous 2 M NaOH solution were mixed in a glass beaker, 0.8 g of Compound I was added while stirring to dissolve it, and then 1.6 g of polyvinylpyrrolidone was added while stirring to dissolve it. This solution was spray dried using a small spray dryer B-290 with an inlet temperature of 50 to 70°C (outlet temperature of 35 to 55°C), an aspirator output of 100%, a peristaltic pump output of 20%, and a spray air flowmeter height of 25 to 30 mm. The powder recovered after spray drying was dried overnight at 60°C in a vacuum dryer, and the resulting powder was used as a solid dispersion.

Table 1 presents the results of evaluating the physicochemical properties of the obtained solid dispersion as follows. A device for measuring X-ray powder diffraction (XRPD) was used to evaluate the crystalline state, high-performance liquid chromatography (HPLC) was used to evaluate the drug content, a device for measuring thermogravity (TG) was used to evaluate the water content, a nuclear magnetic resonance (NMR) spectrometer was used to evaluate the residual solvent, and a device for differential scanning calorimetry (DSC) was used to evaluate the glass transition point.

XRPD results showed a halo pattern characteristic of amorphous material, indicating that Compound I is present in the solid dispersion in an amorphous form. DSC results showed a single and very high glass transition point (184°C). The other properties, drug content, water content, and residual solvents, are shown below. It is noted that when the solid dispersion prepared in the same manner as in this Example was stored for six months under the conditions of 40°C and 30% RH, and then subjected to XRPD measurement, peak for crystallization was not found, and the dispersion had sufficient physical stability.

**[Table 1]**

| Property | Measurement results |
|---|---|
| X-ray diffraction pattern | Amorphous |
| Drug content | 34% (w/w) |
| Water content | 3% (w/w) |
| Residual ethanol | < 600 ppm |
| Glass transition point | 184°C |

### Examples 2 and 3

Examples 2 and 3 describe the cases where sodium cation was used as the cationic species and hydroxypropyl cellulose or methacrylic acid copolymer LD was used as the polymer to form a solid dispersion by the same preparation method as in Example 1.

### [Example 2]

A solid dispersion containing Compound I, hydroxypropyl cellulose (NISSO HPC-SL, Nippon Soda) as a polymer, and sodium cation as a cationic species was prepared. Hydroxypropyl cellulose is a nonionic polymer having a cellulose skeleton, and has a hydroxypropyl group as a functional group. The preparation conditions were the same as in Example 1. (Compound I:polymer weight ratio was 1:2, and the Compound I:sodium molar ratio was 1:1.6)

### [Example 3]

A solid dispersion containing Compound I, methacrylic acid copolymer LD (EUDRAGIT L100-55, EVONIK) as a polymer, and sodium cation as a cationic species was prepared. Methacrylic acid copolymer LD is an ionic (acidic) polymer, and is a copolymer of methacrylic acid and ethyl acrylate. As a spray solvent, a mixture of methanol (Junsei Chemical) and an aqueous 2M NaOH solution (volume ratio of 94.5:5.5) was used, and other conditions were the same as in Example 1. (Compound I:polymer weight ratio was 1:2, and the Compound I:sodium molar ratio was 1:1.8)

### Comparative examples 1 and 2

Comparative examples 1 and 2 describe cases in which the form of Compound I as an active pharmaceutical ingredient was changed. Salt formation (free form crystal to salt crystal) and amorphization (free form crystal to amorphous free form) are widely known as means for improving solubility, and solubility improvements were attempted using these forms.

### [Comparative example 1]

This comparative example describes the besylate salt of Compound I. Compound I (4.0g) was dissolved in a mixture of acetic acid (40 mL) and aqueous 2 mol/L benzenesulfonic acid solution (3.78mL) at 80°C. To this solution, methyl ethyl ketone (20 mL) was added, and the mixture was stirred for ten minutes, cooled to 40°C, and then stirred for ten minutes. After cooling to room temperature, methyl ethyl ketone (20 mL) was added and the mixture was stirred for ten minutes. Further addition of methyl ethyl ketone (40 mL) was followed by filtration and drying of the precipitated solids. The dried solids (4.17 g) were suspended in methyl ethyl ketone (60 mL) and stirred at 40°C for two hours. Furthermore, adding acetic acid (2 mL) and water (2 mL) and stirring at 40°C for 17 hours, yielded crystals which were filtered and dried to obtain the besylate crystals of Compound I (3.75 g).

### [Comparative example 2]

This comparative example describes the amorphous Compound I (amorphous free form). In a mixture of tetrahydrofuran (Junsei Chemical) and water (volume ratio 85:15) at room temperature, Compound I was dissolved at a concentration of about 4%. The obtained solution was spray dried using a small spray dryer B-290 with an inlet temperature of 50 to 70°C (outlet temperature of 35 to 55°C), aspirator output of 100%, peristaltic pump output of 20%, and spray air flowmeter height of 25 to 30 mm. The powder recovered after spray drying was dried overnight at room temperature in a vacuum dryer to obtain the amorphous free form. The obtained powder was confirmed to be amorphous by XRPD.

### Comparative examples 3 to 5

Comparative examples 3 to 5 describe the formation of a solid dispersion formed with Compound I and a polymer. A solid dispersion wherein drugs are dispersed in a polymer matrix in an amorphous state is widely known as a means for improving solubility other than the means mentioned above, and solubility improvements were attempted using this form.

### [Comparative example 3]

This comparative example describes the formation of a solid dispersion containing Compound I and polyvinylpyrrolidone as a polymer. Compound I and polyvinylpyrrolidone at a weight ratio of 1:2 (33.3% compound and 66.7% polymer) were dissolved at room temperature in a mixture of tetrahydrofuran and methanol (volume ratio 70:30) at about 12% solid content concentration (Compound I concentration of 4%). Next, the obtained solution was spray dried using a small spray dryer B-290. The spray drying conditions were the same as in Comparative example 2. The powder recovered after spray drying was dried overnight at 60°C in a vacuum dryer to obtain the solid dispersion. The obtained powder was confirmed to be amorphous by XRPD.

### [Comparative example 4]

This comparative example describes the formation of a solid dispersion containing Compound I and hydroxypropyl cellulose as a polymer. The preparation conditions were the same as in Comparative example 3 (Compound I:polymer weight ratio was 1:2). The obtained powder was confirmed to be amorphous by XRPD.

### [Comparative example 5]

This comparative example describes the formation of a solid dispersion containing Compound I and hypromellose acetate succinate (Shin-Etsu AQOAT-LF, Shin-Etsu Chemical) as a polymer. Hypromellose acetate succinate is an ionic (acidic) polymer with a cellulose skeleton, and has acetyl and succinoyl groups as functional groups. A mixture of tetrahydrofuran and water (volume ratio of 85:15) was used as the spray solvent and the other conditions were the same as in Comparative example 3 (Compound I:polymer weight ratio was 1:2). The obtained powder was confirmed to be amorphous by XRPD.

### Comparative example 6

Comparative example 6 describes a case where a cationic species was added during the amorphization of Compound I indicated in Comparative example 2, that is, a case where no polymer was added when compared to Examples 1 to 3.

### [Comparative example 6]

This comparative example describes the formation of an amorphous substance containing Compound I and a sodium cation as a cationic species. Compound I was dissolved at room temperature in a mixture of ethanol and an aqueous 2 M NaOH solution (volume ratio of 95:5) at a Compound I concentration of about 4%. The obtained solution was spray dried using a small spray dryer B-290 at an inlet temperature of 50 to 70°C (outlet temperature of 35 to 55°C), aspirator output of 100%, peristaltic pump output of 20%, and spray air flowmeter height of 25 to 30 mm (Compound I:sodium molar ratio of 1:1.6). The powder recovered after spray drying was dried overnight at room temperature in a vacuum dryer. The obtained powder was confirmed to be amorphous by XRPD.

### Comparative example 7

Comparative example 7 describes a case where a polyvinylpyrrolidone polymer was physically mixed with the same components as in Comparative example 6, or more specifically, a case where the composition was the same as in Example 1, but Compound I and the polymer did not form a solid dispersion.

### [Comparative example 7]

The same components as in Comparative example 6 (amorphous Compound I containing sodium (Compound I: sodium molar ratio of 1:1.6)) and polyvinylpyrrolidone were weighed to adjust the ratio of Compound I and the polymer to a weight ratio of 1:2 (33.3% compound and 66.7% polymer), and this was mixed using a mortar and pestle.

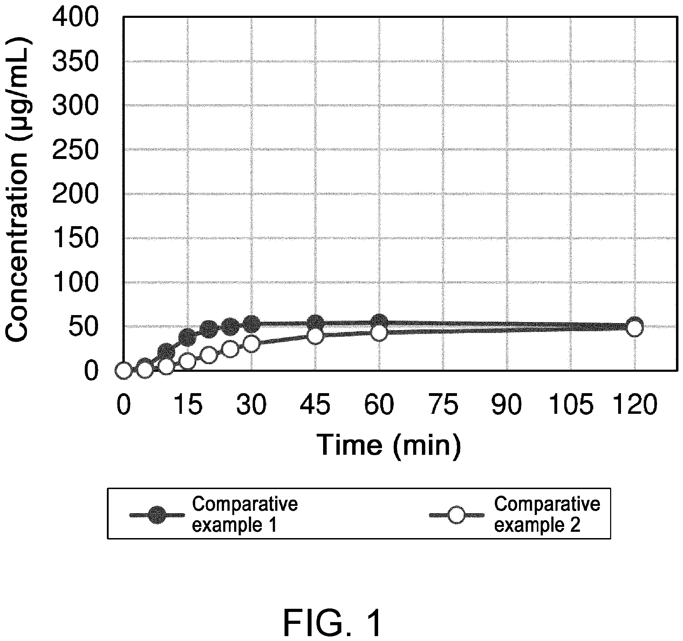

The results of performing dissolution tests on the salt crystals, amorphous materials, and solid dispersions prepared in Comparative examples 1 to 7 and the solid dispersions of the present application prepared in Examples 1 to 3 are shown in Figs. 1 to 3. To 50 mL of artificial simulated intestinal fluid (fasted-state simulated intestinal fluid (FaSSIF)) warmed to 37°C, a sample was charged so that 20 mg of Compound I would be added (compound concentration of 400 µg/mL). While stirring this mixture using a small dissolution tester VK7010 (Varian) at a paddle rotation speed of 50 rpm, samples were taken over time (5, 10, 15, 20, 25, 30, 45, 60, and 120 minutes), these were filtered through a 0.45-µm PVDF filter, and Compound I concentration in the obtained filtrates was quantified by HPLC. It is noted that, each of the same samples was measured at n = 2 to 4, and the average values (average concentration) ± standard deviation are shown in the figure.

Furthermore, for the purpose of evaluating the solubility of the type I crystal of the free form of Compound I in FaSSIF, an excess amount of the type I crystal of the free form of Compound I was placed in FaSSIF, and after soaking and stirring at 37°C for 24 hours or more, this was subjected to centrifugation. When Compound I concentration in the separated supernatant was quantified by HPLC, the solubility was found to be 1 µg/mL. In addition, the solubility of the type II crystal of the free form of Compound I evaluated in the same manner was 2 µg/mL.

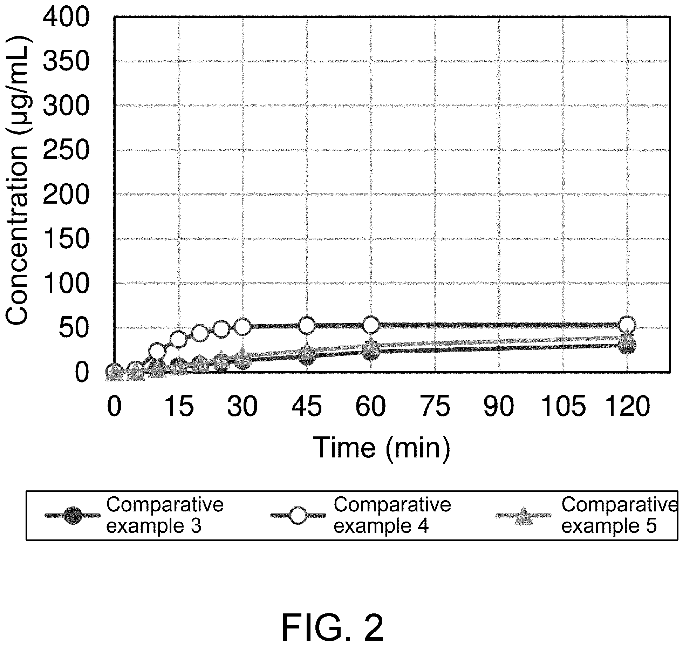

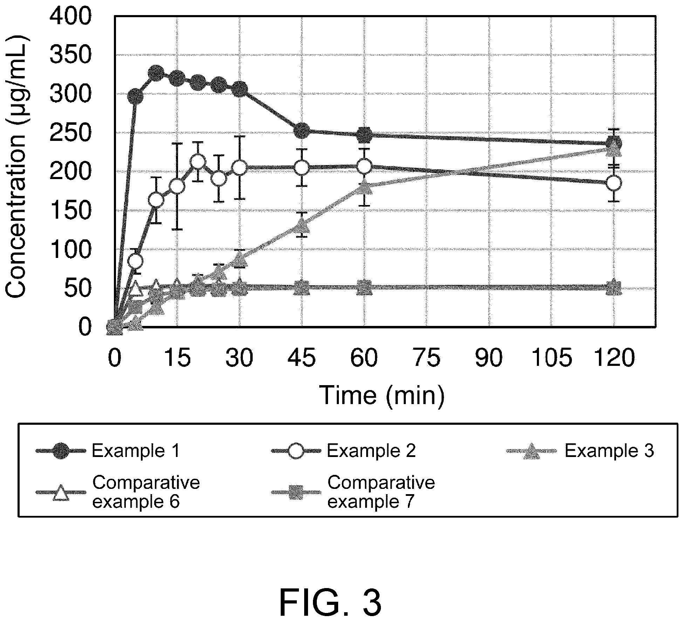

According to the dissolution test results, the solubility at 120 minutes was at most about 50 µg/mL in any of Comparative examples 1 and 2 (Fig. 1) and Comparative examples 3 to 5 (Fig. 2). This means that the solubility was confirmed to be improved by about 50 times compared to that of the type I crystal of the free form. Meanwhile, the results of the solid dispersions of Examples 1 to 3 (Fig. 3) showed much higher solubility. At 120 minutes, they showed at most 8 times higher solubility than those in Comparative examples 3 to 5, which correspond to the conventional solid dispersion; in addition, they showed solubility improvement effect of at most 236 times compared to the solubility of the type I crystal of the free form. Furthermore, although Comparative example 3 and Example 1 used the same polymer component, and Comparative example 4 and Example 2 used the same polymer component, solubilities were 4 to 8 times higher for the solid dispersions of the present Examples. Moreover, in Fig. 3, the solubility results in Comparative example 6, which is the case for a solid dispersion containing only sodium, and in Comparative example 7, which involves physically mixing the polymer, were comparable to those of Comparative examples 1 to 5. In these cases, the two-component system formed with the polymer or cationic species could not show the high effect seen in the present Examples, and high dissolution improvement effects were observed only when the three components constituting the requirements were combined in the solid dispersion.

### Examples 4 to 6 and Comparative example 8

Examples 4 to 6 and Comparative example 8 describe cases where a surfactant was added to the solid dispersion. Using sodium cation as the cationic species, and hydroxypropyl cellulose, copovidone, and hypromellose acetate succinate as the polymer, a solid dispersion was formed using the same preparation method as in Example 1, and then sodium lauryl sulfate serving as the surfactant was combined.

### [Example 4]

A solid dispersion containing Compound I, hydroxypropyl cellulose as a polymer, and sodium cation as a cationic species was prepared as in Example 1 (Compound I:polymer weight ratio of 1:2, Compound I:sodium molar ratio of 1:1.6). The obtained solid dispersion and sodium lauryl sulfate (NIKKOL SLS, Nikko Chemicals) were weighed and mixed with a mortar and pestle so that the mixing ratio of sodium lauryl sulfate becomes half the amount of Compound I (Compound I:polymer:sodium lauryl sulfate = 1:2:0.5) (weight ratio)).

### [Example 5]

A solid dispersion containing Compound I, copovidone (Kollidon VA64, BASF) as a polymer, and sodium cation as a cationic species was prepared as in Example 1 (Compound I:polymer weight ratio of 1:2, Compound I:sodium molar ratio of 1:1.6). Copovidone is a copolymer of N-vinyl-2-pyrrolidone and vinyl acetate, and is a nonionic polymer. Sodium lauryl sulfate was weighed and added to the obtained solid dispersion, so that its mixing ratio would be half the amount of Compound I (Compound I:polymer: sodium lauryl sulfate = 1:2:0.5) (weight ratio)), and this was mixed with a mortar and pestle.

### [Example 6]

A solid dispersion containing Compound I, hypromellose acetate succinate as a polymer, and sodium cation as a cationic species was prepared. This was dissolved at room temperature in a mixture of tetrahydrofuran, ethanol, water, and an aqueous 0.5 M NaOH solution (Wako Pure Chemicals) (weight ratio of 50:20:20:10) at a solid content concentration of about 6% (2% in terms of Compound I concentration). The other conditions applied were the same as in Example 1 (Compound I: polymer weight ratio of 1:2, Compound I: sodium molar ratio of 1:1.6). Sodium lauryl sulfate was weighed and added to the obtained solid dispersion, so that its mixing ratio would be half the amount of Compound I (Compound I:polymer: sodium lauryl sulfate = 1:2:0.5) (weight ratio)), and this was mixed with a mortar and pestle.

### [Comparative example 8]

A solid dispersion containing Compound I and a sodium cation as the cationic species (Compound I:sodium molar ratio of 1:1.6) was prepared as in Comparative example 6. Sodium lauryl sulfate was weighed and added to the obtained solid dispersion, so that its mixing ratio would be half the amount of Compound I (Compound I: sodium lauryl sulfate = 1:0.5) (weight ratio)), and this was mixed using a mortar and pestle.

### Dissolution test results

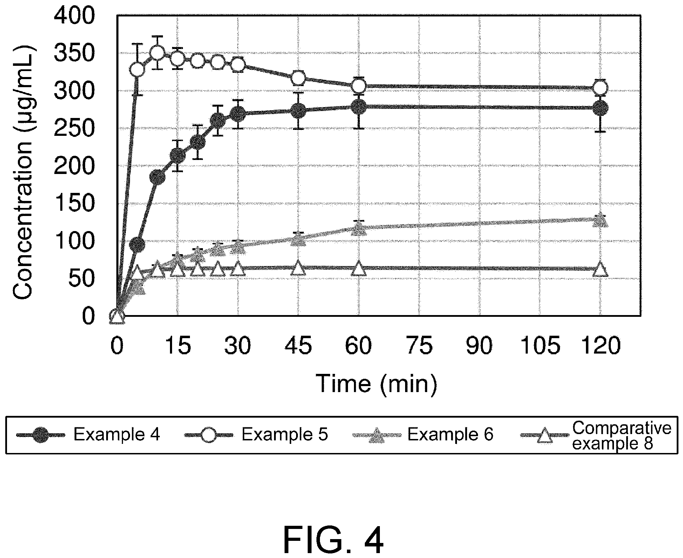

The results of performing dissolution tests on the surfactant-containing solid dispersions prepared in Examples 4 to 6 and Comparative example 8 are shown in Fig. 4. It is noted that only the peak derived from sodium lauryl sulfate was observed by XRPD in all samples, and Compound I was confirmed to be amorphous. As a result of dissolution, at the 120-minute time point, about 2 to 5 times higher solubility was shown in the cases of Examples 4 to 6 where sodium lauryl sulfate was added to a polymer-containing solid dispersion, as compared to Comparative example 8 where sodium lauryl sulfate was added to a solid dispersion not containing a polymer. Therefore, adding a surfactant to the aforementioned three-component solid dispersion to improve the wettability of the powder and such was shown to be possible (without impairing the high solubility).

### Examples 7 and 8

Examples 7 and 8 describe cases where a solid dispersion was formed using sodium cation as the cationic species and polyvinylpyrrolidone as the polymer, at a compound:polymer ratio different from that of Example 1.

### [Example 7]

A solid dispersion containing Compound I, polyvinylpyrrolidone as a polymer, and sodium cation as a cationic species was prepared. Compound I and the polymer at a weight ratio of 1:1.5 (40% compound and 60% polymer) were dissolved at room temperature in a mixture of ethanol and an aqueous 2 M NaOH solution (volume ratio of 95:5) at about 10% solid content concentration (4% in terms of Compound I concentration). The other conditions were the same as in Example 1 (Compound I:sodium molar ratio of 1:1.6).

### [Example 8]

A solid dispersion containing Compound I, polyvinylpyrrolidone as a polymer, and sodium cation as a cationic species was prepared. Compound I and the polymer at a weight ratio of 1:1(50% compound and 50% polymer) were dissolved at room temperature in a mixture of ethanol and an aqueous 2 M NaOH solution (volume ratio of 95:5) at about 8% solid content concentration (4% in terms of Compound I concentration). The other conditions were the same as in Example 1 (Compound I:sodium molar ratio of 1:1.6).

### Dissolution test results

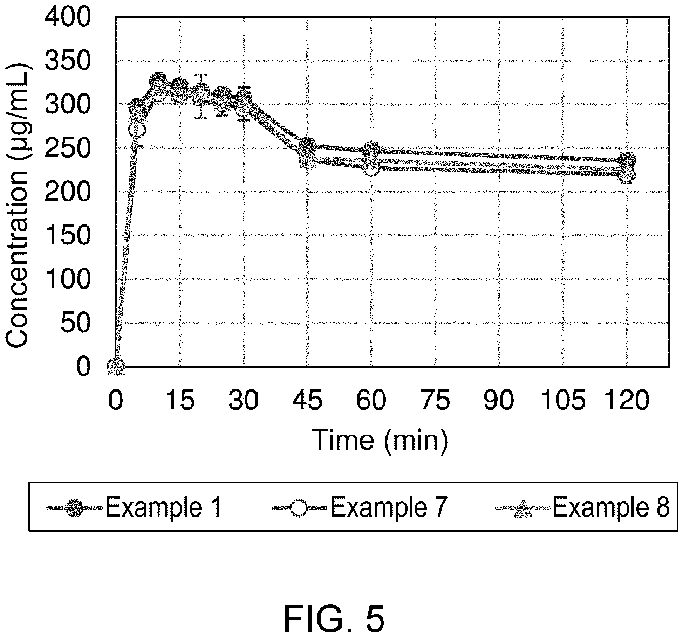

The results of performing dissolution tests on the solid dispersions prepared in Examples 7 and 8 and results of Example 1 used as a comparison are shown in Fig. 5. All samples were confirmed to be amorphous by XRPD. Even when the compound:polymer weight ratio was changed, the results showed high solubility as in Example 1.

### Examples 9 and 10

Examples 9 and 10 describe cases where a solid dispersion was formed using sodium cation as the cationic species and polyvinylpyrrolidone as the polymer, at a different molar ratio of sodium than that of Example 7. Except in Example 3 where the sodium molar ratio was 1.8, spray solutions were prepared in Examples 1 to 8 at sodium molar ratio of 1.6 relative to Compound I, and formation of a solid dispersion with lower proportion of sodium was attempted.

### [Example 9]

A solid dispersion containing Compound I, polyvinylpyrrolidone as a polymer, and sodium cation as a cationic species was prepared. Compound I and the polymer at a weight ratio of 1:1.5 were dissolved at room temperature in a mixture of ethanol and an aqueous 5 M NaOH solution (Wako Pure Chemicals) (volume ratio of 99.7:0.3) at about 2.5% solid content concentration (1% in terms of Compound I concentration) (in the spray solution, the molar ratio of sodium to Compound I was 1.1). The other conditions were the same as in Example 1.

### [Example 10]

A solid dispersion containing Compound I, polyvinylpyrrolidone as a polymer, and sodium cation as a cationic species was prepared. Compound I and the polymer at a weight ratio of 1:1.5 were dissolved at room temperature in a mixture of ethanol and an aqueous 5 M NaOH solution (volume ratio of 98.4:1.6) at about 10% solid content concentration (4% in terms of Compound I concentration) (in the spray solution, the molar ratio of sodium to Compound I was 1.3). The other conditions were the same as in Example 1.

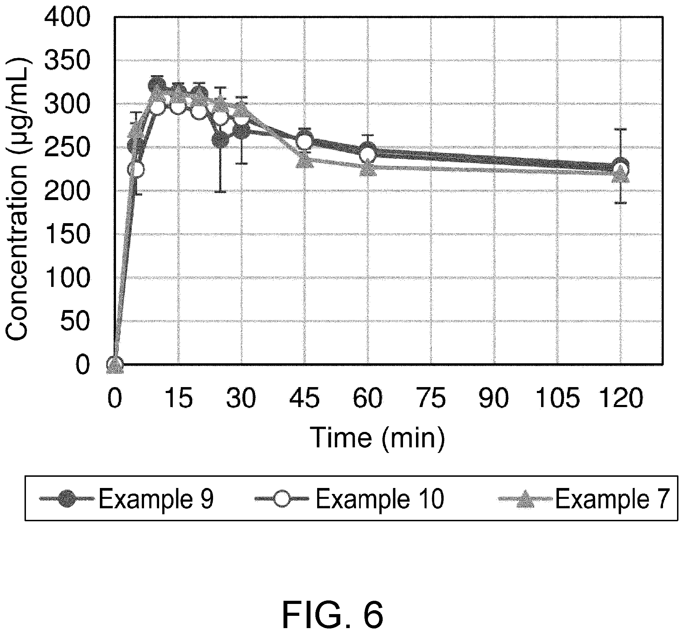

For the solid dispersions prepared in Examples 7, 9, and 10, sodium quantification was performed by ion chromatography, and the compound quantification was performed by HPLC. As a result, all of the solid dispersions were confirmed to contain sodium therein at the molar ratio set for preparation. Furthermore, the results of performing dissolution tests on these solid dispersions are shown in Fig. 6. The results indicated high solubility, same as in the previous Examples, even when the molar ratio of sodium was changed. It is noted that the obtained powder was confirmed to be amorphous by XRPD.

### Examples 11 to 14

Examples 11 to 14 describe cases where solid dispersions were formed using various cationic species other than sodium cation. As the cationic species, a potassium cation and an arginine cation were examined.

### [Example 11]

A solid dispersion containing Compound I, polyvinylpyrrolidone as a polymer, and potassium cation as a cationic species was prepared. Compound I and the polymer at a weight ratio of 1:2 were dissolved at room temperature in a mixture of ethanol and an aqueous 1 M KOH solution (Wako Pure Chemicals) (volume ratio of 90:10) at about 12% solid content concentration (4% in terms of Compound I concentration). The other conditions were the same as in Example 1 (Compound I:potassium molar ratio of 1:1.6).

### [Example 12]

A solid dispersion containing Compound I, hydroxypropyl cellulose as a polymer, and potassium cation as a cationic species was prepared. Compound I and the polymer at a weight ratio of 1:2 were dissolved at room temperature in a mixture of ethanol and an aqueous 1 M KOH solution (volume ratio of 90:10) at about 12% solid content concentration (4% in terms of Compound I concentration). The other conditions were the same as in Example 1 (Compound I: potassium molar ratio of 1:1.6).

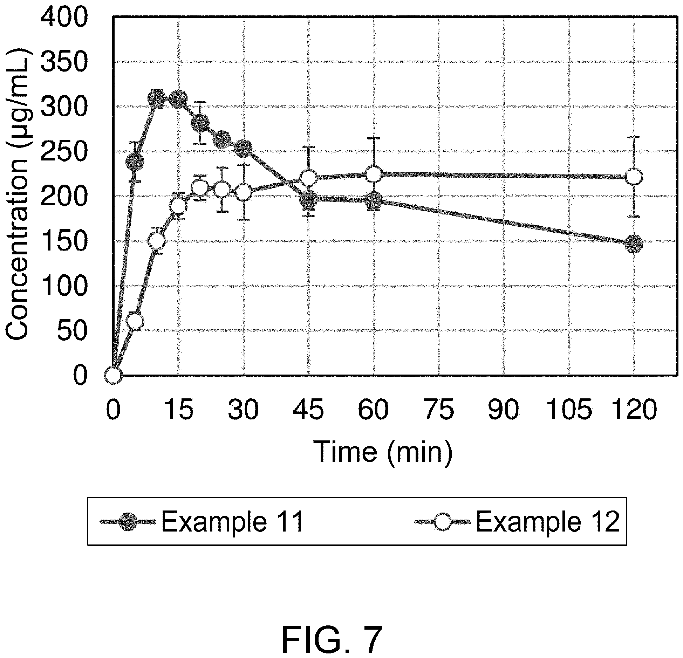

Results of performing dissolution tests on the solid dispersions prepared in Examples 11 and 12 are shown in Fig. 7. The results indicated that even when potassium cations were used as the cationic species, the solubility was higher compared to the solubility of two-component solid dispersions not containing cationic species, such as those in Comparative examples 3 to 6.

### Examples 13 and 14

Examples 13 and 14 describe cases where solid dispersions were formed using arginine cation as a cationic species. In these Examples, rather than preparation by the aforementioned spray drying method, preparation by hot melt extrusion was attempted.

### [Example 13]

A solid dispersion containing Compound I, copovidone as a polymer, and arginine cation as a cationic species was prepared. Compound I, L-arginine (Wako Pure Chemicals), and the polymer, physically mixed in a mortar at a weight ratio of 1:1:8, were placed into a small twin-screw hot-melt extruder ZE-9 (Three-Tec). The mixture was subjected to hot melt extrusion at a condition of screw rotation speed 20 rpm, and barrel temperatures (H1/H2/H3 = 150/220/210°C). The obtained rod-shaped sample was roughly crushed using a sample mill, and the obtained granular powder was made into a solid dispersion (Compound I:arginine molar ratio of 1:3.6).

### [Example 14]

A solid dispersion containing Compound I, polyvinyl alcohol (Parteck MXP, MERCK) as a polymer, and arginine cation as a cationic species was prepared. Polyvinyl alcohol is a nonionic polymer of vinyl alcohol. Other conditions were the same as in Example 13 (Compound I:polymer weight ratio of 1:8, Compound I:arginine molar ratio of 1:3.6).

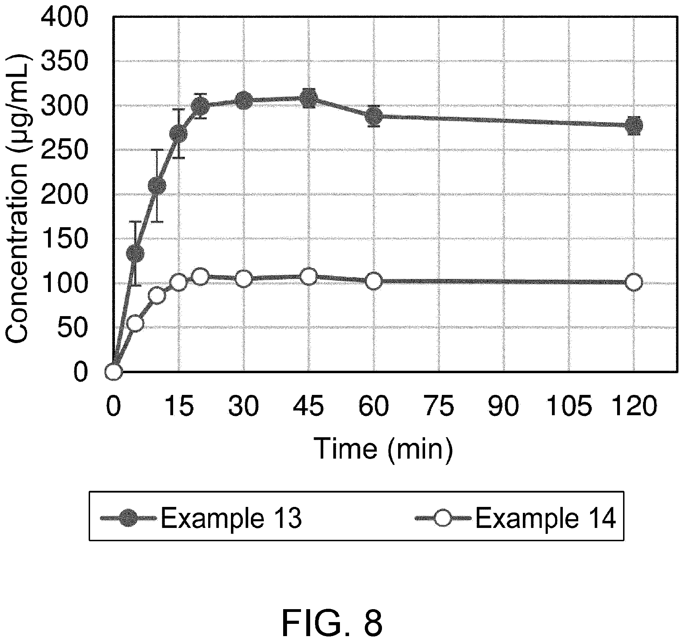

Results of performing dissolution tests on the solid dispersions prepared in Examples 13 and 14 are shown in Fig. 8. The results indicated that even when arginine cation was used as the cationic species, the solubility was higher compared to the solubility of two-component solid dispersions containing no cationic species such as those in Comparative examples 3 to 6. In addition, the results showed that the solid dispersion can be prepared by not only the spray drying method but also by hot melt extrusion.

### [Example 15] In vivo absorbability test

This Example describes the results of an *in vivo* (beagle dog) test performed for Examples that showed high dissolution in *in vitro* dissolution tests. Exposure (Cₘₐₓ and AUC) to Compound I was evaluated for oral administration of compositions such as those described below.

Composition A: A gelatin capsule (size # 00) filled with besylate crystals equivalent to that of Comparative example 1 and various additives that do not assist dissolution was used.
Composition B: A gelatin capsule (size # 00) filled with solid dispersions equivalent to that of Example 1 was used.
Composition C: A gelatin capsule (size # 00) filled with solid dispersions equivalent to that of Example 2 was used.
Composition D: A gelatin capsule (size # 00) filled with solid dispersions equivalent to that of Example 5 mixed with sodium lauryl sulfate was used.

The animal species used was a 12- to 18-month old beagle dog (male), and as pretreatment, 0.25 mL/kg of pentagastrin (Sigma-Aldrich) at a dose of 6 µg/kg was administered intramuscularly in the hind limbs, one hour before starting the oral administration. Capsules containing the composition were orally administered to fasted animals whose residual food was removed at 17:00 on the day before administration, and then using a catheter the animals were subjected to forced oral administration of tap water at 50 mL/animal and flushing with 10 mL of air. Blood (0.6 mL) was collected from the forearm cephalic vein before administration and at 0.083, 0.5, 1, 2, 4, 7, and 24 hours after administration. The collected blood was immediately centrifuged (12,000 rpm), and the concentration of Compound I in the obtained plasma was quantified by HPLC-MS/MS.

Table 2 shows the dose (dose), n number (number of dogs used), and results (Cₘₐₓ, AUC_{inf}, and bioavailability) of the *in vivo* test. That is, Table 2 shows the results of an *in vivo* (beagle dog) test performed using Examples 1, 2, and 5 and Comparative example 1, and shows the exposure of Compound I when each composition was orally administered (Cₘₐₓ, AUC, and bioavailability).

Here, Cₘₐₓ is the observed maximum concentration of Compound I in plasma, and AUC_{inf} is the area under the curve of Compound I concentration in plasma versus time. These values are the average regarding the number of dogs subjected to the administration. Bioavailability was calculated as a percentage (%) of AUC_{inf} divided by dose with respect to that obtained from a 2 mg/kg intravenous administration given to separate dog groups.

**[Table 2]**

| Composition | Dose (mg/kg) | n number | Cₘₐₓ (ng/mL) | AUC_{inf} (ng·h/mL) | Bioavailability (%) |
|---|---|---|---|---|---|
| A (Comparative example 1) | 64 | 6 | 3240 | 16500 | 12 |
| B (Example 1) | | 4 | 8550 | 59300 | 44 |
| C (Example 2) | | | 4450 | 31600 | 23 |
| D (Example 5) | | | 8440 | 61200 | 45 |

From the test results, the groups to which solid dispersions of the Examples were administered exhibited higher absorbability (exposure) compared to the group to which the composition containing the besylate crystals of Compound I was administered, and an improvement of about 1.4 to 2.6 times for Cₘₐₓ and an improvement of 1.9 to 3.7 times for AUC_{inf} were observed. This indicates that the present solid dispersions not only improve *in vitro* dissolution, but also improves *in vivo* bioavailability.

### [Production example 1]: Production of type I crystal of the free form of Compound I

Compound I (1 g) was suspended in a mixture of ethanol (8 mL) and water (2 mL), and stirred at 75°C for three hours. After cooling the suspension to 45°C, it was stirred for another day. The obtained crystals were filtered and dried to obtain the type I crystals of Compound I (0.9 g).

The type I crystals had excellent storage stability.

### [Production example 2]: Production of type II crystal of the free form of Compound I

Compound I was dissolved in 10 times its amount (v/w) of dimethyl sulfoxide, dispensed into vials, and then freeze-dried. To the solid obtained by freeze-drying, five times its amount (v/w) of 1-butanol was added, and the mixture was stirred for one week to obtain the type II crystals of Compound I.

The XRPD of the crystals obtained in Production examples 1 and 2 was measured under the following conditions:
Measuring device: SmartLab (manufactured by Rigaku)
Anticathode: Cu
Tube voltage: 45 kV
Tube current: 200 mA
Scanning range: 5° to 35°
Sampling width: 0.02°

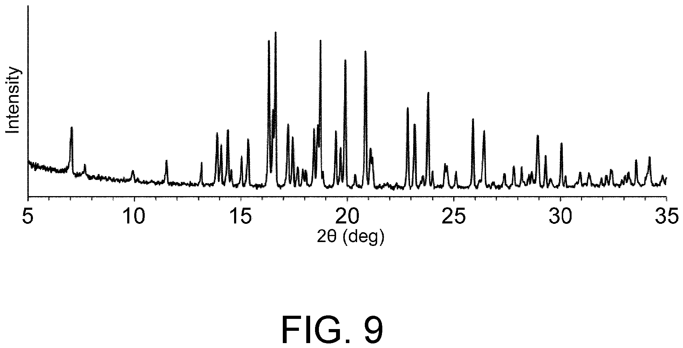

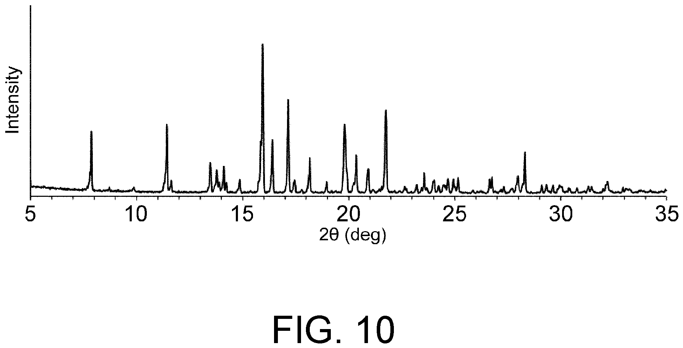

Fig. 9 shows the measurement results of the type I crystal, and Fig. 10 shows the measurement results of the type II crystal.

### [Industrial Applicability]

The solid dispersion of the present invention has enabled great improvement of solubility and accompanying improvement of absorbability in the digestive tract of 1-(3,5-dimethyl-4-(2-((4-oxo-2-(4-(trifluoromethoxy)phenyl)-1,3,8-triazaspiro[4.5]deca-1-en-8-yl)sulfonyl)ethyl)phenyl)-5,5-dimethylimidazolidine-2,4-dione, a poorly water-soluble compound having strong PTH-like effects and high metabolic stability.

## Claims

1. A solid dispersion which comprises Compound I having the following structural formula: 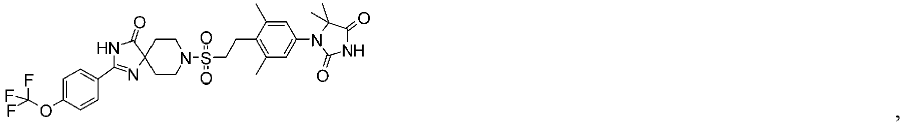 a pharmaceutically acceptable polymer, and a pharmaceutically acceptable cationic species.

2. The solid dispersion of claim 1, which comprises Compound I existing in an amorphous form.

3. The solid dispersion of claim 1 or 2, wherein the pharmaceutically acceptable polymer is at least one polymer selected from the group consisting of polyvinylpyrrolidone, copovidone, polyvinyl alcohol, cellulosic polymer, and methacrylic acid copolymer.

4. The solid dispersion of claim 1 or 2, wherein the pharmaceutically acceptable polymer is at least one polymer selected from the group consisting of polyvinylpyrrolidone, copovidone, polyvinyl alcohol, hydroxypropyl cellulose, hypromellose acetate succinate, and methacrylic acid copolymer LD.

5. The solid dispersion of any one of claims 1 to 4, wherein the pharmaceutically acceptable cationic species is at least one cationic species supplied by a base having a pKa value of 11 or higher.

6. The solid dispersion of any one of claims 1 to 5, wherein the pharmaceutically acceptable cationic species is at least one selected from the group consisting of a sodium cation, potassium cation, and arginine cation.

7. The solid dispersion of any one of claims 1 to 6, wherein the weight ratio of Compound I to the polymer in the composition is about 1:9 to about 1:1.

8. The solid dispersion of any one of claims 1 to 7, wherein the weight ratio of Compound I to the polymer in the composition is about 1:2 to about 1:1.

9. The solid dispersion of any one of claims 1 to 8, wherein the molar ratio of the cationic species to Compound I in the composition is 0.8 or higher.

10. The solid dispersion of any one of claims 1 to 9, which further comprises a surfactant, wherein the surfactant is a pharmaceutically acceptable surfactant.

11. The solid dispersion of any one of claims 1 to 10, wherein Compound I is derived from:
type I crystal of the free form: a crystal of Compound I whose powder X ray diffraction pattern comprises peaks at diffraction angles, represented by 2θ, of 14.4°, 15.3°, 16.6°, and 18.7° (±0.2°); and/or
type II crystal of the free form: a crystal of Compound I whose powder X ray diffraction pattern comprises peaks at diffraction angles, represented by 2θ, of 7.9°, 13.5°, 15.9°, and 21.8° (±0.2°).

12. A pharmaceutical composition which comprises the solid dispersion of any one of claims 1 to 11.

13. A method for producing a solid dispersion which comprises the steps of:
(i) providing Compound I having the following structural formula: 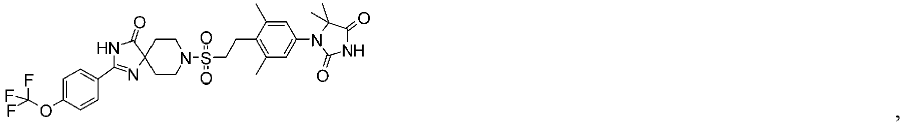 a pharmaceutically acceptable polymer, and a base that yields a pharmaceutically acceptable cationic species;
(ii) mixing the Compound I, the pharmaceutically acceptable polymer, and the base that yields the pharmaceutically acceptable cationic species; and
(iii) obtaining a solid dispersion comprising the Compound I, the pharmaceutically acceptable polymer, and the pharmaceutically acceptable cationic species.

14. The method of claim 13, wherein the steps (ii) and (iii) comprise the following steps, respectively:
(ii-a) preparing a mixed solution by mixing the Compound I, the pharmaceutically acceptable polymer, and the base that yields the pharmaceutically acceptable cationic species in a solvent; and
(iii-a) removing the solvent from the mixed solution obtained in the step (ii-a).

15. The method of claim 14, wherein the step (iii-a) comprises the step of removing the solvent and drying by spray drying.

16. The method of any one of claims 13 to 15, wherein in the step (i), Compound I is derived from:
type I crystal of the free form: a crystal of Compound I whose powder X ray diffraction pattern comprises peaks at diffraction angles, represented by 2θ, of 14.4°, 15.3°, 16.6°, and 18.7° (±0.2°); and/or
type II crystal of the free form: a crystal of Compound I whose powder X ray diffraction pattern comprises peaks at diffraction angles, represented by 2θ, of 7.9°, 13.5°, 15.9°, and 21.8° (±0.2°).
